# EUROPEAN PATENT SPECIFICATION

(11) **EP 4 125 608 B1**
(45) Date of publication and mention of the grant of the patent: **29.04.2026**
(21) Application number: 21714704.0
(22) Date of filing: 12.03.2021
(51) Int. Cl.: A61B 8/08, A61B 8/00

(54) **PATIENT MONITOR, PHYSIOLOGICAL INFORMATION DISPLAY METHOD AND PROGRAM**
PATIENTENMONITOR, VERFAHREN ZUR ANZEIGE PHYSIOLOGISCHER INFORMATIONEN UND PROGRAMM
DISPOSITIF DE SURVEILLANCE DE PATIENT, PROCÉDÉ D'AFFICHAGE D'INFORMATIONS PHYSIOLOGIQUES ET PROGRAMME

(30) Priority: 26.03.2020 JP 2020056709
(43) Date of publication of application: 08.02.2023
(73) Proprietor: Nihon Kohden Corporation, Shinjuku-ku Tokyo 161-8560 (JP)
(72) Inventor: OURA, Mitsuhiro, Tokorozawa-shi, Saitama 359-0037 (JP); KOBAYASHI, Naoki, Tokorozawa-shi, Saitama 359-0037 (JP)
(74) Representative: Grünecker Patent- und Rechtsanwälte PartG mbB
(86) International application number: PCT/JP2021/010233
(87) International publication number: WO 2021/193170

(56) References cited:
- US-A- 5 951 476
- US-A1- 2018 214 117
- US-A1- 2018 344 182

## Description

### [Technical Field]

The presently disclosed subject matter relates to a patient monitor, a physiological information display method, and a program.

### [Background Art]

In clinical practice, various techniques are used to display a physiological waveform which is acquired via a sensor to grasp a state of a subject.

Patent Literature 1 discloses a technique of displaying a physiological waveform related to brain tissue pulsation based on a signal acquired via an ultrasonic wave transducer.

### [Citation List]

### [Patent Literature]

[PTL 1]Patent Literature 1: JP-T-2017-523889
US 5,951,476 discloses a method for detecting intracranial microbleeding by continuing microshifting of a person's brain within the cranium.

### [Summary of Invention]

### [Technical Problem]

As illustrated in FIG. 5, Patent Literature 1 discloses that a physiological waveform corresponding to intracranial pressure is displayed on a screen. However, in order to grasp an intracranial situation in more detail, it is more preferable to grasp a relationship between a depth and a motion in a body of a subject.

The presently disclosed subject matter has been made in view of such room for improvement, and an object thereof is to provide a patient monitor, a physiological information display method, and a program that provide a screen by which the relationship between the depth and the motion in the body of the subject can be easily grasped.

### [Solution to Problem]

The present invention provides a patient monitor according to claims 1 and 16, an ultrasonic wave processing method according to claim 17 and a computer program according to claim 18.

According to the presently disclosed subject matter, since the relationship between the depth and the motion is displayed, a user can easily grasp the relationship between the depth and the motion at the depth. As a result, the user can easily grasp an abnormal portion, and grasp a difference between the abnormal portion and a normal portion.

### [Effects of the Invention]

According to the presently disclosed subject matter, the patient monitor, the physiological information display method, and the program that provide the screen by which the relationship between the depth and the motion in the body of the subject can be easily grasped can be provided.

### [Brief Description of Drawings]

FIG. 1 is a schematic view illustrating a human intracranial structure.
FIGS. 2A and 2B are conceptual diagrams illustrating, in terms of a waveform, a motion of brain tissue (brain lateral pulsation and brain stem) at a certain intracranial position.
FIG. 3 is a conceptual diagram illustrating a configuration of a physiological information processing system 1.
FIG. 4 is a block diagram illustrating the configuration of the physiological information processing system 1.
FIG. 5 illustrates a concept of measurement performed by an ultrasonic wave transducer 100.
FIG. 6 illustrates a display example of a patient monitor 10.
FIG. 7 illustrates a display example of the patient monitor 10.
FIG. 8 illustrates a display example of the patient monitor 10.
FIG. 9 illustrates a display example of the patient monitor 10.
FIG. 10 illustrates a display example of the patient monitor 10.
FIG. 11 illustrates a display example of the patient monitor 10.
FIG. 12 illustrates a display example of the patient monitor 10.
FIG. 13 illustrates a display example of the patient monitor 10.
FIG. 14 illustrates a display example of the patient monitor 10.
FIG. 15 illustrates a display example of the patient monitor 10.
FIG. 16 illustrates a display example of the patient monitor 10.
FIG. 17 illustrates a display example of the patient monitor 10.
FIG. 18 illustrates a display example of the patient monitor 10.
FIG. 19 illustrates a display example of the patient monitor 10.
FIG. 20 illustrates a display example of the patient monitor 10.
FIG. 21 is a conceptual diagram illustrating a modification of the configuration of the physiological information processing system 1 according to Embodiment 1.
FIGS. 22A and 22B illustrate display examples of the patient monitor 10.
FIGS. 23A and 23B illustrate display examples of the patient monitor 10.
FIGS. 24A to 24C illustrate display examples of the patient monitor 10.
FIGS. 25A and 25B illustrate a display examples of the patient monitor 10.

### [Description of Embodiments]

### <Embodiment 1>

Hereinafter, an embodiment of the presently disclosed subject matter will be described with reference to the drawings. First, ultrasonic wave measurement and brain tissue handled by a physiological information processing system 1 that includes a patient monitor 10 of the present embodiment will be described.

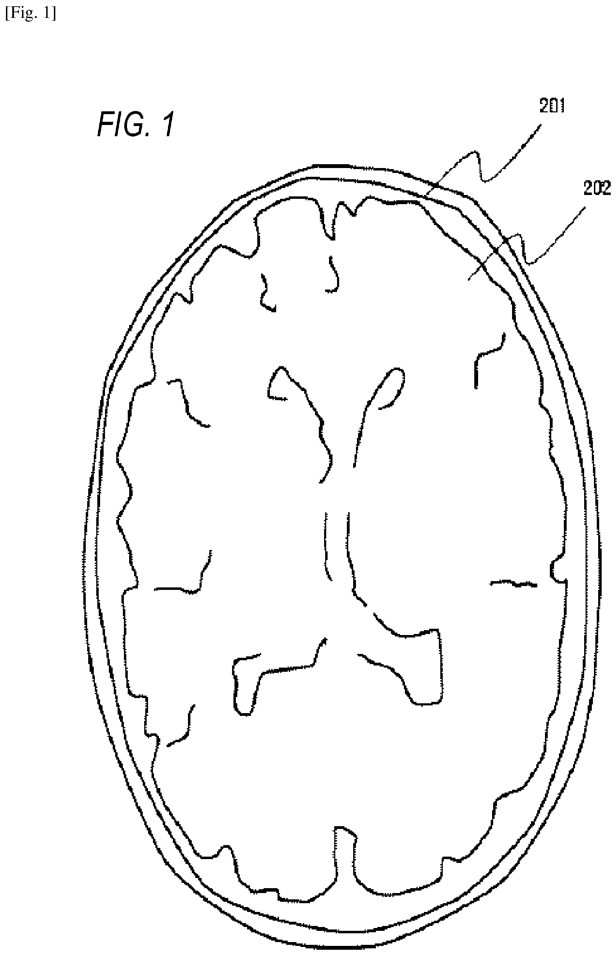

First, a mechanism of a brain and pulsation of brain tissue will be described. As illustrated in FIG. 1, in a human head, a brain 202 (solid), blood, and medullary fluid are accommodated in an internal space covered with a cranial bone 201. Intracranial pressure (ICP), which is pressure inside the cranial bone, is generated due to an influence of the brain, the blood and the medullary fluid. The intracranial pressure (ICP) is measured when there is suspicion of various diseases. Examples of such diseases include stroke, osmotic metabolic convulsion, coma with unknown cause, hydrocephalus, and head injury. It is also clear that ICP measurement is useful for evaluation of severe headache, walking disorder, urinary incontinence, and dementia.

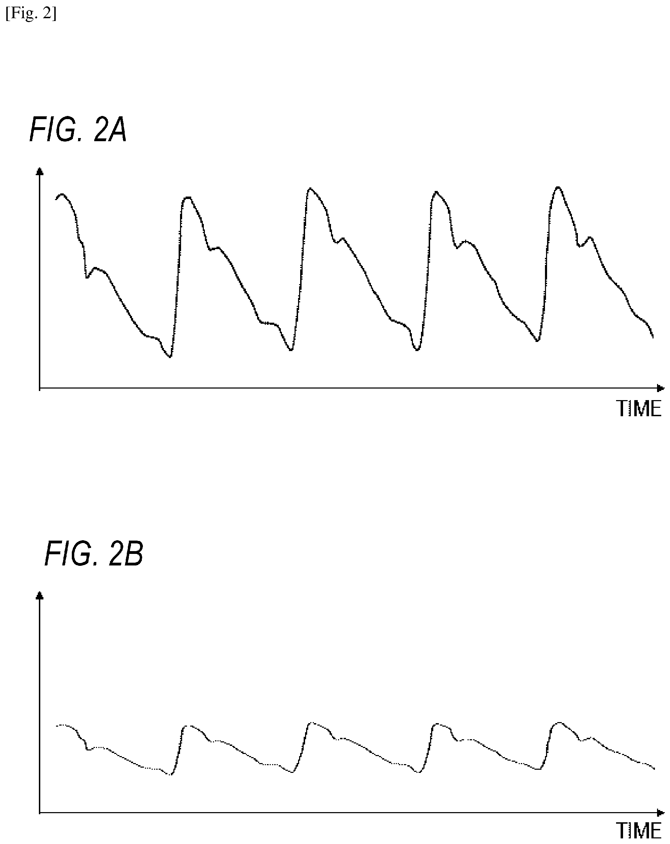

FIGS. 2A and 2B illustrate, in terms of a waveform, a motion of brain tissue (brain lateral pulsation and brain stem) at a certain intracranial position. As illustrated in FIG. 2A, amplitude is generated by a motion caused by a heartbeat. In a case where the brain tissue is normal, pulsation is generated due to circulation of blood caused by heartbeats. A waveform of such pulsation can be acquired at each intracranial depth. A shape of the pulsation waveform changes in accordance with a state of a brain of a subject (swelling of entire brain tissue, swelling of a part of the brain tissue, and the like) and the intracranial depth.

ICP can be enhanced due to various factors. For example, ICP can be enhanced due to abnormal outflow of cerebrospinal fluid (CSF), which is a cause of the hydrocephalus, or swelling of solid tissue (cerebral edema, intracranial hematoma, tumor, and the like). ICP monitoring is generally achieved by measurement using a pressure gauge inserted into the cranial bone 201 of a patient. However, an applicant has proposed an alternative method of non-invasive ICP monitoring using an ultrasonic wave transducer. For details, refer to Patent Literature 1.

Increase in cerebrospinal fluid (CSF), an amount of intracranial blood, or brain tissue causes ICP enhancement. A state where both the brain and the cerebrospinal fluid (CSF) are accommodated in the cranial bone is referred to as a green status. A state where the brain is swollen to such an extent that a part of the brain is abutted against the cranial bone while the cerebrospinal fluid (CSF) surrounding the brain temporarily disappears is referred to as an orange status. A state where the brain swells and substantially fills inside of a calvarium while fluids filling a ventricle located in the brain are falsely removed is referred to as a blue status. Further, a state where the brain stem starts to extend from a normal position and the ICP is significantly enhanced is referred to as a red status. For details, refer to FIGS. 2a to 2c and paragraphs 0033 and 0034 of Patent Literature 1.

Such status can be specified by using a transcranial brain tissue Doppler method (TCBTD) based on lateral pulsation of the brain and a motion of the brain stem at a plurality of depths. In the transcranial brain tissue Doppler method, echo luminance of solid brain tissue is about 30 dB (1000 times) higher than echo luminance of the blood. Therefore, an ultrasonic wave pulse can be transmitted by the ultrasonic wave transducer, and a reflected wave (echo) can be received through a thick region of the cranial bone.

When swelling occurs in a part of the brain due to an influence of symptoms, solid tissue (brain) is distorted, so that regions having different pressure are generated in the cranial bone. For example, motions of brain tissue (lateral pulsation of the brain and the brain stem), and waveforms indicating ICP values and ICP changes over time are different between a portion where the solid tissue is swollen due to an influence of tumor and any other portion. Therefore, it is easier to realize appropriate diagnosis and treatment for the subject when not only pulsation (and hence ICP) at a single position can be grasped, but pulsation (and hence ICP) at various depths at different distances from a body surface can also be grasped. The patient monitor 10 described below provides a technique for grasping information of pulsation at each depth in a non-invasive manner.

Next, a configuration of the patient monitor 10 according to the present embodiment will be described. The patient monitor 10 according to the present embodiment displays, on a screen, a physiological waveform based on a reflected wave of an ultrasonic wave acquired from an ultrasonic wave transducer 100.

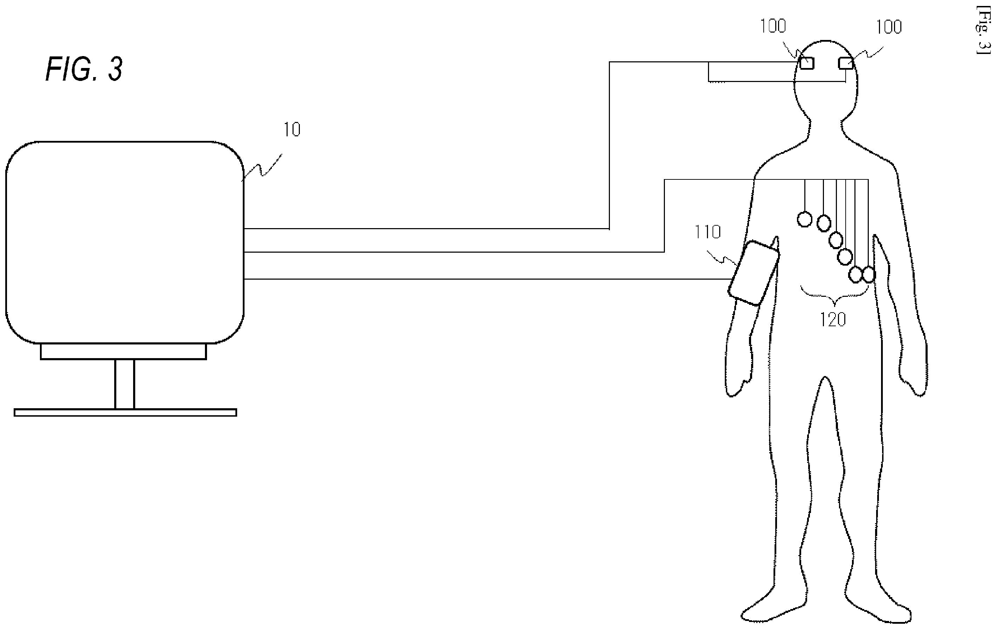

FIG. 3 illustrates a configuration of the physiological information processing system 1 according to the present embodiment. The physiological information processing system 1 includes the patient monitor 10 and various sensors (the ultrasonic wave transducer 100, a cuff 110, and an electrocardiogram electrode 120) which are connectable to the patient monitor 10. As illustrated in the drawing, the subject is attached with the various sensors (the ultrasonic wave transducer 100, the cuff 110, and the electrocardiogram electrode 120). The patient monitor 10 processes signals acquired from the various sensors so as to display measurement waveforms and measurement values of various vital signs.

The ultrasonic wave transducer 100 of the patient monitor 10 illustrated in the drawing acquires a reflected wave indicating a motion (brain tissue pulsation) generated at each depth by an ultrasonic wave irradiated at a plurality of depths in the body of the subject. Then the patient monitor 10 displays a change over time in an index value of the motion at each depth of the subject based on the reflected wave corresponding to each depth. Here, the patient monitor 10 displays the change over time in the index value in terms of (A) map information expressing a magnitude of the index value on a map taking depth on a first axis and elapsed time on a second axis, by a change in at least one of a hue, lightness and chroma. Such display control will be described later.

The patient monitor 10 is preferably a bedside monitor configured to display various types of physiological information (such as electrocardiogram, non-invasive blood pressure, and respiration) of a patient (subject) in addition to brain tissue pulsation. The patient monitor 10 may also be a patient monitor having a defibrillation function, or may be a tablet PC type device or a wearable device (for example, AR glasses or the like) worn on a body (for example, an arm or a head) of a medical worker. In the present embodiment, it is assumed that the patient monitor 10 is a bedside monitor configured to display brain tissue pulsation (information indicating the index value calculated from the reflected wave).

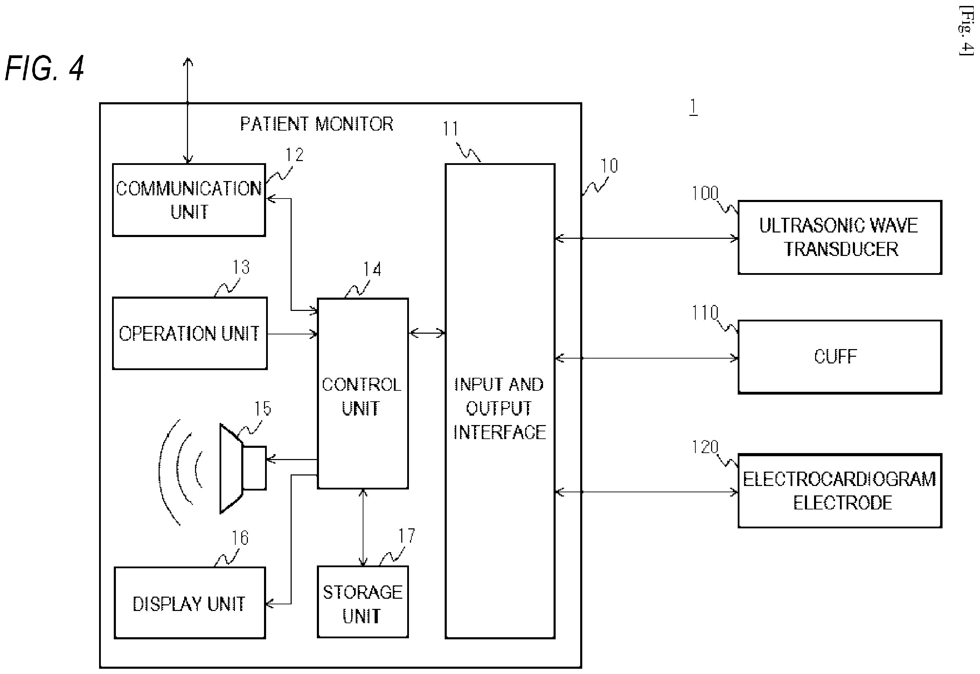

FIG. 4 is a block diagram illustrating an internal configuration of the physiological information processing system 1 according to the present embodiment. As illustrated in the drawing, the patient monitor 10 includes an input and output interface 11, a control unit 14, a storage unit 17, a communication unit 12, a display unit 16, and an operation unit 13. The patient monitor 10 is configured to be connected to the ultrasonic wave transducer 100. The patient monitor 10 may also be connectable to the various sensors that acquire physiological information, and in this example, the patient monitor 10 is connected to the cuff 110 that measures blood pressure and the electrode 120 that measures the electrocardiogram.

The ultrasonic wave transducer 100 is not necessarily connected to the patient monitor 10 by wire as long as the ultrasonic wave transducer 100 is capable of transmitting and receiving data to and from the patient monitor 10, and the ultrasonic wave transducer 100 may also be wirelessly connected thereto via any wireless communication standard as desired (Bluetooth (registered trademark), Wi-Fi (registered trademark), or the like). The same also applies to connection between the other sensors and the patient monitor 10.

The ultrasonic wave transducer 100 is fixed so as to be in contact with a position on a body surface of a head where the motion (pulsation) of the brain tissue of the subject can be acquired, for example, a forehead or a temple of the subject. The ultrasonic wave transducer 100 may be any ultrasonic wave transducer as long as the ultrasonic wave transducer 100 can transmit and receive the ultrasonic wave to and from a living body (a periphery of the brain) of the subject. For example, the ultrasonic wave transducer 100 may be a disposable stick-on sheet-shaped sensor which is capable of transmitting and receiving the ultrasonic wave to and from the living body. The ultrasonic wave transducer 100 may also be a reusable probe fixed by a biocompatible mesh tape, which is capable of transmitting and receiving the ultrasonic wave to and from the living body. A plurality of the ultrasonic wave transducers 100 may be worn by the subject, for example, fixed to left and right temples.

The ultrasonic wave transducer 100 transmits the ultrasonic wave to each depth inside and outside a cranial bone, and receives an ultrasonic wave (reflected wave) reflected at each depth. That is, the ultrasonic wave transducer 100 acquires information in which the depth and the reflected wave are associated with each other. The reflected wave indicates the motion (pulsation) of the brain tissue at each depth. The pulsation can be changed due to a motion of an arteriole, artery or vein in the brain, and the motion can be changed at a site where swelling of the brain tissue occurs. By acquiring such pulsation over time (continuously), a physiological waveform, which indicates an intracranial motion at a certain depth over time, is obtained. Such a physiological waveform is highly related to the intracranial pressure. Hereinafter, a description of the reflected wave includes (does not distinguished between) a state of raw data (analog data) and a state of digital data obtained after A/D conversion performed by the control unit 14 to be described later below or the like.

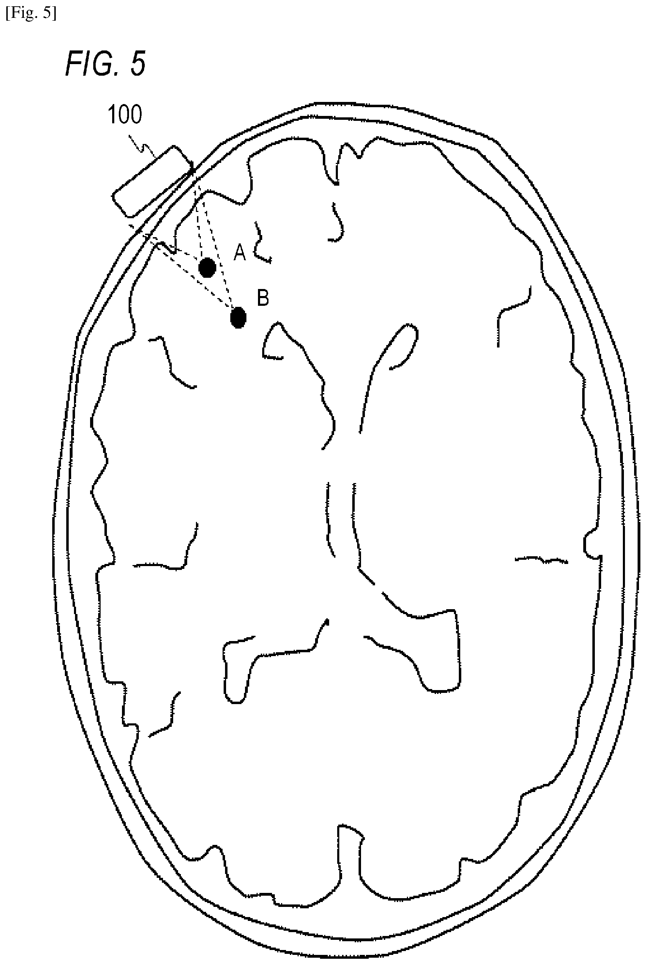

A concept of measurement performed by the ultrasonic wave transducer 100 will be described with reference to FIG. 5. The ultrasonic wave transducer 100 irradiates each point of a cranium with an ultrasonic wave. In this example, the ultrasonic wave transducer 100 irradiates the ultrasonic wave to point A and point B in the cranium. Then the ultrasonic wave transducer 100 receives the reflected wave from each point (point A and point B). Then the ultrasonic wave transducer 100 associates information on a depth from the body surface with the reflected wave and transmits the associated information and reflected wave to the patient monitor 10. Although the ultrasonic wave transducer 100 irradiates point A and point B with the ultrasonic wave in this example for clarification of the description, the reflected wave may be acquired at any location. Of course, it is also possible for the ultrasonic wave transducer 100 to actually irradiate the ultrasonic wave from the body surface of 0 mm to any distance as desired (for example, 50 mm) in the body without any gap therebetween, and acquire the reflected wave in association with the depth from the body surface.

FIG. 4 will be referred to again. The input and output interface 11 (an example of an acquisition unit that acquires physiological signals of various physiological parameters including the reflected wave of the ultrasonic wave) acquires the reflected wave acquired at each depth by the ultrasonic wave transducer 100. That is, the input and output interface 11 acquires the reflected wave which is acquired at each depth around the brain by the ultrasonic wave transducer 100. The ultrasonic wave transducer 100 may also convert the reflected wave into digital data by performing A/D conversion or the like thereon, and then transmit the digital data to the input and output interface 11. The input and output interface 11 may transmit various control signals (control of gain and the like) to the ultrasonic wave transducer 100 under control of the control unit 14.

The control unit 14 includes one or more memories and one or more processors. The one or more memories are configured to store computer readable commands (programs). The one or more memories may each be configured with a read-only memory (ROM) in which various programs and the like are stored, and a random access memory (RAM) including a plurality of work areas in which various programs and the like executed by the one or more processors are stored. The one or more memories may also be constituted by flash memories. The one or more processors each include, for example, at least one of a central processing unit (CPU) and a graphics processing unit (GPU). The one or more processors may each include a field-programmable gate array (FGPA) and/or an application specific integrated circuit (ASIC). The CPU may be configured with a plurality of CPU cores. The GPU may be configured with a plurality of GPU cores. The one or more processors may be configured to load a physiological information program specified from various programs incorporated in the storage unit 17 or the ROM onto the RAM and execute various processes in cooperation with the RAM.

The storage unit 17 is, for example, a storage device (storage) such as a hard disk drive (HDD), a solid state drive (SSD), or a flash memory, and is configured to store programs and various types of data.

The control unit 14 performs appropriate signal processing on various signals acquired via the input and output interface 11 and then writes the signals in the storage unit 17. For example, the control unit 14 performs processing (for example, A/D conversion) which is necessary for the reflected wave acquired by the ultrasonic wave transducer 100 at each depth in the vicinity of the brain, and then appropriately writes the reflected wave in the storage 17 and reads the reflected wave as appropriate. The control unit 14 writes and reads other physiological information (for example, a blood pressure signal and a respiratory signal) and the like to and from the storage unit 17 in the same manner as a general patient monitor.

The communication unit 12 is configured to connect the patient monitor 10 to a communication network. The communication unit 12 may include various wired connection terminals used for communication with an external server, a central monitor, or the like via the communication network (for example, a LAN, a WAN, or the Internet). The communication unit 12 may also include a communication processing circuit, such as an RF circuit used for performing wireless communication with an access point (for example, a wireless LAN router or a wireless base station), and a transmission and reception antenna. A wireless communication standard between the access point and the patient monitor 10 is, for example, Wi-Fi (registered trademark), Bluetooth (registered trademark), ZigBee (registered trademark), LPWA, or fifth generation mobile communication system (5G).

The display unit 16 may be a display device such as a liquid crystal display or an organic EL display. The display unit 16 may also be a display device such as a transmissive or non-transmissive head mount display, or an augmented reality (AR) display that is provided as a separate housing from the patient monitor 10 and is mounted on a head of an operator. Further, the display unit 16 may be a projector device that projects an image on a screen or the like. The patient monitor 10 may also not include the display unit 16. In this case, after the patient monitor 10 transmits image data to an external display device in a wireless or wired manner, the image display device may display the image data transmitted from the patient monitor 10.

The operation unit 13 is configured to receive an input operation of a medical worker who operates the patient monitor 10 and to generate an instruction signal corresponding to the input operation. The operation unit 13 is, for example, a touch panel arranged on the display unit 16 in an overlapping manner, a mouse or keyboard connected to the patient monitor 10, and/or a physical operation button arranged in a housing of the patient monitor 10. After the instruction signal generated by the operation unit 13 is transmitted to the control unit 14, the control unit 14 performs a predetermined operation in accordance with the instruction signal.

The control unit 14 controls pressure of the cuff 110 in the same manner as a normal patient monitor so as to measure non-invasive blood pressure of the subject. Specifically, the control unit 14 controls acquisition of a blood pressure signal from the cuff 110, analysis of the blood pressure signal, display of a blood pressure value and a blood pressure waveform on the display unit 16, and the like.

Same or similarly, the control unit 14 acquires an electrocardiogram signal from the electrocardiogram electrode 120, performs various analyses, and controls display of an electrocardiogram waveform on the display unit 16 in the same manner as the normal patient monitor. Although not illustrated, the control unit 14 may also measure respiration, SpO2, and the like in the same manner as the normal patient monitor.

The control unit 14 performs alarm control by a method same or similar to that of the normal patient monitor, and sounds alarm sounds (upper and lower limit alarms, technical alarms, and the like) via a speaker 15 if necessary.

Next, generation of the image data and the display control performed by the control unit 14 will be described in detail. The input and output interface 11 acquires the reflected wave, which is generated by the ultrasonic wave irradiated to the plurality of depths in the body of the subject by the ultrasonic wave transducer 100 and indicates the position and the motion (brain tissue pulsation) of each depth. The input and output interface 11 and the ultrasonic wave transducer 100 may also perform various types of signal processing on the reflected wave.

The control unit 14 calculates the index value of the motion at each depth of the subject based on the reflected wave corresponding to each depth, and displays the change over time in the calculated index value. Here, the patient monitor 10 displays the change over time in the index value in terms of (A) map information expressing a magnitude on a map taking depth on a first axis and elapsed time on a second axis, by change in at least one of hue, lightness and chroma. The display control performed by the control unit 14 is a concept not only includes displaying the image data on the display unit 16 provided on the housing of the patient monitor 10 (or the display unit 16 detachably provided on the patient monitor 10), but also transmitting the image data to an external device to perform display on the external device.

Here, the index value of the motion at each depth is, for example, intensity of the reflected wave, frequency of the reflected wave, a displacement amount calculated based on the reflected wave, displacement velocity or displacement acceleration calculated from the displacement amount, magnitude and frequency of amplitude calculated from the displacement amount, or the like.

When the intensity of the reflected wave is used as the index value, the control unit 14 may calculate signal strength of the reflected wave by a known signal processing method. When the frequency of the reflected wave is used as the index value, the control unit 14 may calculate the frequency by performing frequency conversion on the reflected wave.

When the displacement amount is used as the index value, the control unit 14 may perform IQ demodulation on the reflected wave, extract an I signal component and a Q signal component, plot the I signal component and the Q signal component on an IQ plane, and uses a declination thereof to calculate the displacement amount. The control unit 14 may also use another type of signal processing (for example, signal processing that calculates the displacement amount from a change in a feature point position of the signal strength) on the reflected wave to calculate the declination and then calculate the displacement amount.

When the displacement velocity or the displacement acceleration is used as the index value, the control unit 14 may calculate the displacement velocity or the displacement acceleration by performing general processing such as differentiation or second order differentiation on the displacement amount calculated as described above.

When the magnitude of the amplitude is used as the index value, the control unit 14 cuts out a beat from the calculated displacement amount. For example, the control unit 14 may detect a peak point from a waveform indicating a change in the displacement amount in the same manner as in a normal electrocardiogram analysis, and cut out one beat from the peak point to another peak point (a waveform from a previous beat of the heart to a current beat). Then the control unit 14 may calculate the magnitude of the amplitude by subtracting a maximum value and a minimum value of the waveform within the one beat.

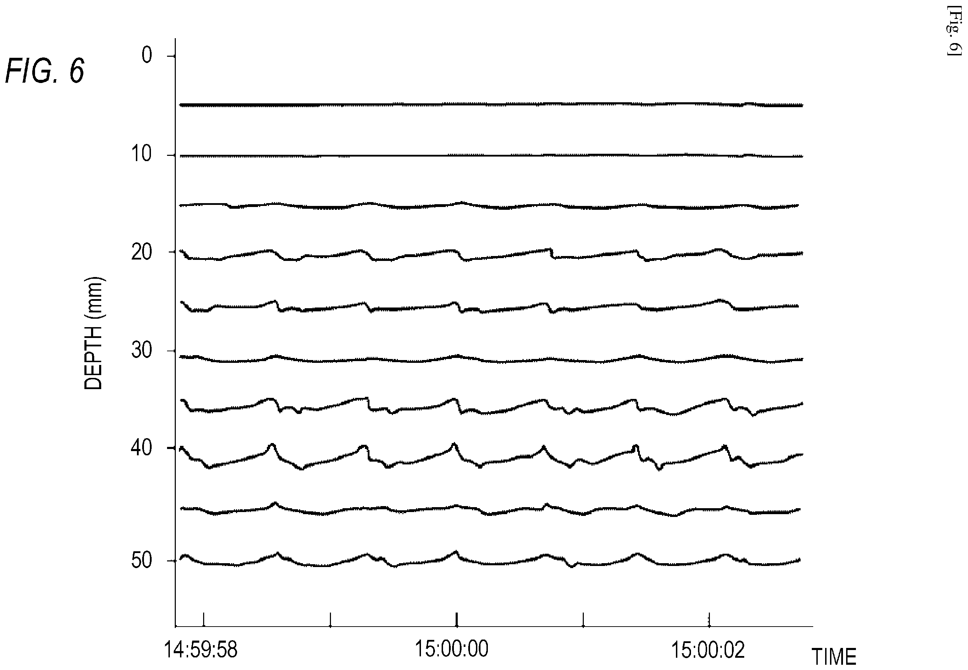

The control unit 14 displays the change over time in the index value corresponding to each depth in terms of the expression (A). In an alternative example, a format of the "plurality of waveforms corresponding to each depth" will be described with reference to FIG. 6. FIG. 6 illustrates an example of the image data displayed in terms of "a plurality of waveforms corresponding to each depth", and illustrates the change over time in the displacement amount (one type of the index value).

The control unit 14 calculates the displacement amount at each depth as the index value based on the reflected wave at each depth, and displays, on the display unit 16, image data in which the change over time in the displacement amount at each depth is depicted on two-dimensional coordinates. The two-dimensional coordinates (two-dimensional graph) indicate the depth in the body of the subject (depth from the body surface) on a vertical axis (first axis) and indicates times on a horizontal axis (second axis). The horizontal axis may also indicate, for example, an elapsed time from a start of the measurement. That is, the horizontal axis may indicate any information as long as a lapse of time can be grasped thereby.

In the example of FIG. 6, waveforms (waveforms indicating changes in the displacement amount) are displayed at intervals of 5 mm from a distance of 5 mm from the body surface. That is, waveforms of depths of 5 mm / 10 mm / 15 mm / 20 mm / 25 mm / 30 mm / 35 mm / 40 mm / 45 mm / 50 mm from the body surface are displayed. That is, the control unit 14 calculates the displacement amount (an amount of motion at the depth) as the index value from the reflected wave at the depth of 5 mm / 10 mm / 15 mm / 20 mm / 25 mm / 30 mm / 35 mm / 40 mm / 45 mm / 50 mm from the body surface, and displays the change in the displacement amount at each depth as the plurality of waveforms.

The user may be capable of specifying whether to display other physiological parameters (blood pressure and the like) by operating the operation unit 13 provided on the patient monitor 10 or a remote controller (not illustrated). The control unit 14 generates the image data in accordance with information on the specification.

The user may explicitly specify the number of waveforms to be displayed on the two-dimensional graph. The user specifies the number of waveforms by operating the operation unit 13 (for example, a touch panel, a button, or a knob on the patient monitor 10) or the like. The control unit 14 determines a depth that serves as a display target in accordance with the specified number of waveforms. For example, in a case where measurement from the depth of 0 mm to 40 mm is performed and the number of waveforms is specified to be five, the control unit 14 determines that waveforms at the depths of 0 mm, 10 mm, 20 mm, 30 mm, and 40 mm are display targets. Although it is desired that the control unit 14 determines the display targets in such a manner that the depths are at equal intervals, the control unit 14 may also determine the depths serving as the display targets in consideration of other factors, such as a state of the index value (for example, the magnitude of the amplitude). Then the control unit 14 displays the waveform at each depth, which is determined to be the display target, by the same method as in FIG. 6. Since the number of displayed waveforms can be specified in this manner, the waveform can be displayed in such a manner that the user feels easy to see on a screen.

The control unit 14 may change a display mode of the waveform in accordance with the depth. The display mode (format) refers to a thickness, a line type (such as a dotted line, a one-dot chain line, a two-dot chain line, or a solid line), a color, a shape of an end point, or the like. For example, the control unit 14 changes colors according to the depths such that a color of the waveform at the depth of 0 mm to 20 mm is red, a color of the waveform at the depth of 21 mm to 40 mm is green, and a color of the waveform at the depth of 41 mm to 60 is blue. The thickness, the line type, and the shape of the end point may also be changed in the same manner according to the depths. Of course, it is also possible to change both the thickness and the color of the waveform in accordance with the depths. By displaying the waveform whose display mode is changed according to the depths, the user can easily grasp a pulsation state while grasping the depth of the waveform referred to.

The control unit 14 may change the display mode of the waveform based on magnitude of the index value of each waveform. For example, the control unit 14 may change the display mode (format) of the waveform based on the magnitude of the amplitude of the waveform indicating the displacement amount. Hereinafter, a processing example will be described.

The control unit 14 calculates the magnitude of the amplitude of each depth. For example, the control unit 14 may cut out the most recent 10 beats from the waveform of the displacement amount and calculate an average of magnitude of amplitudes of the 10 beats. Then the control unit 14 changes and displays the display mode (thickness, line type, color, shape of end point, and the like) of the waveform according to the magnitude. For example, the control unit 14 may indicate a target waveform by a dotted line when the magnitude of the amplitude is equal to or higher than a predetermined threshold value, and may indicate the target waveform by a solid line when the magnitude of the amplitude is less than the predetermined threshold value. The control unit 14 may also set levels for the magnitude of the amplitude (for example, the amplitude between value A and value B belongs to level 1, the amplitude between value B and value C belongs to level 2, and so on), and may change colors of lines according to the levels. As described above, since the display mode (format) of the waveform is changed in accordance with the magnitude of the amplitude, the user can grasp magnitude of an objective pulsation (amplitude) at each depth. By changing the display mode in accordance with the magnitude of the amplitude in this manner, the user can grasp at a glance a depth where the amplitude is large, a depth where the amplitude is small, a location where the amplitude changes, and the like.

The control unit 14 may also use an index value other than the magnitude of the amplitude to change the display mode of the waveform. For example, the control unit 14 calculates an average value of displacement acceleration of the most recent 10 beats at each depth, and performs level classification of magnitude of the displacement acceleration for each depth. Then the control unit 14 may change the display mode of the waveform at each depth in accordance with levels of the displacement acceleration.

Next, a format of above (A), "map information expressing a magnitude of the index value on a map taking depth on a first axis and elapsed time on a second axis, by a change in at least one of hue, lightness and chroma", will be described.

The map information indicates the depth in the body of the subject (depth from the body surface) on a vertical axis (first axis) and indicates the times on a horizontal axis (second axis) in the same manner as the above "a plurality of waveforms corresponding to each depth". The control unit 14 displays, on two-dimensional coordinates, image data in which the magnitude of the index value of the reflected wave at each depth is expressed by changes in at least one of hue, lightness and chroma. The control unit 14 converts the index value into the hue, lightness or chroma, and uses a result of the conversion to perform depiction. Of course, the horizontal axis may also indicate the elapsed time from the start of the measurement or the like.

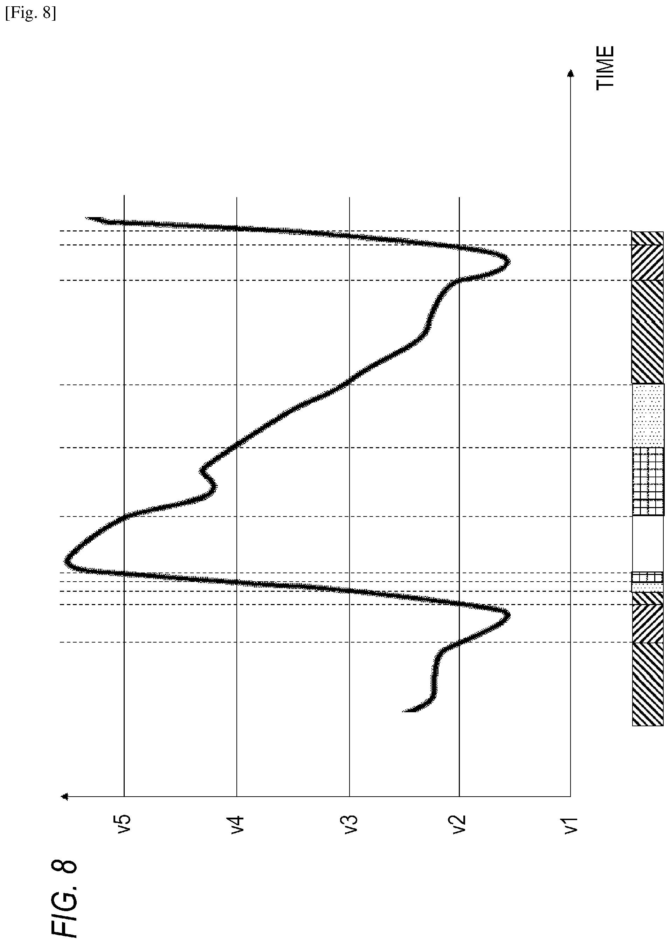

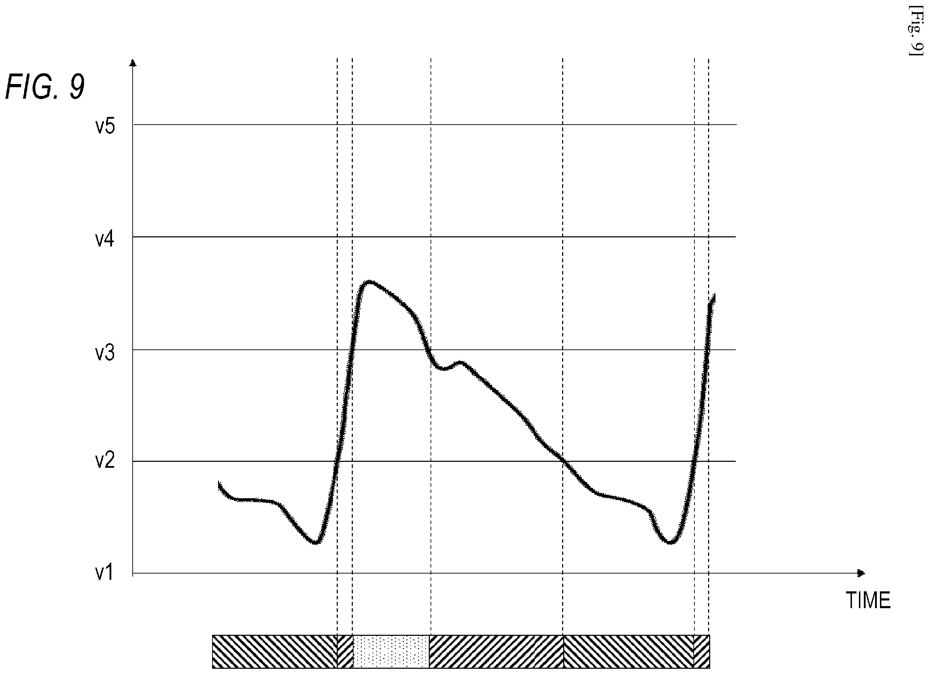

The control unit 14 expresses the magnitude of the index value by changing a color. The change in the color may be achieved by changing at least one of hue, lightness, and chroma. Luminance can also be changed by changing the hue, lightness, or chroma. For example, the control unit 14 maps the index value and the color such that the color is red when the index value is between v1 and v2, and the color is yellow when the index value is between v2 and v3. However, it is more preferable that the control unit 14 performs conversion such that the color changes in a stepwise manner (that is, changes with gradation) in accordance with a change in the index value. Mapping of the pattern is performed by substantially the same method, and details thereof will be described later below with reference to FIGS. 8 and 9.

Of course, the magnitude of the value can be expressed by changing both the color (at least one of hue, lightness, and chroma) and the pattern.

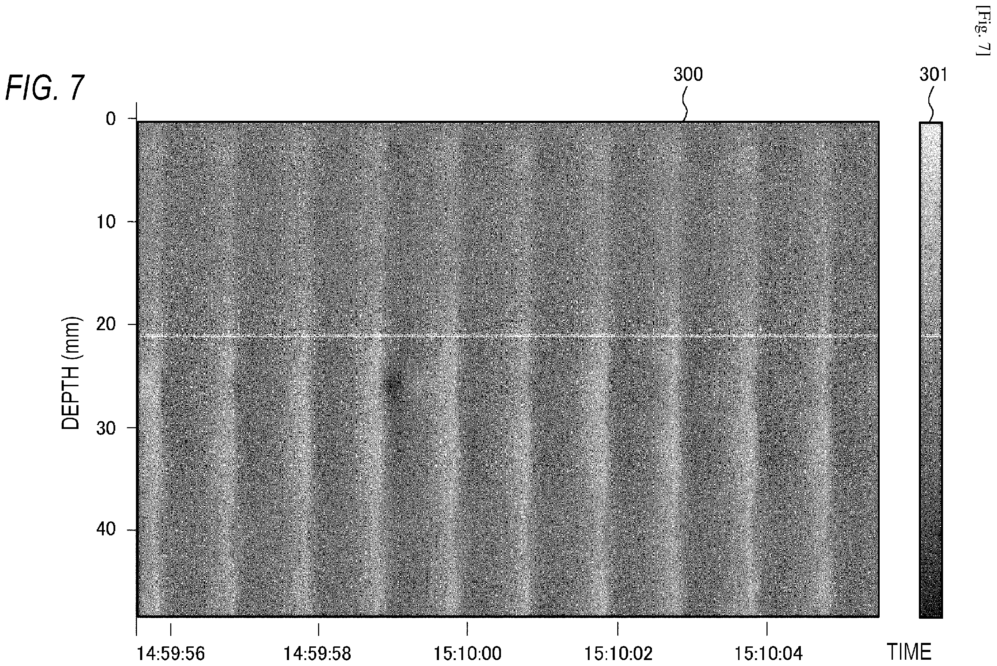

FIG. 7 is an example of map information in which the magnitude of the index value is indicated by the change in the color. In this example, the control unit 14 continuously maps (to colors) and displays the index value of each depth in the vicinity of 0 mm to 50 mm from the body surface. The control unit 14 draws a bar 301 that indicates a scale of the magnitude of the value in addition to an area 300 in which the waveform of each depth is converted into a color and described. By referring to the map information illustrated in FIG. 7, the user (medical worker) can grasp the magnitude of pulsation at each depth, a depth where a trend of the amplitude is displaced, and the like. By continuously handling depths and changes in colors, the user can easily grasp a depth location where a change in the pulsation state is large and grasp an overall pulsation state.

FIGS. 8 and 9 illustrate a concept of indicating the magnitude of the index value of each depth by changes in patterns. In this example, a range between value v1 and value v2 is mapped to hatching of diagonal lines from an upper left side to a lower right side, a range between value v2 and value v3 is mapped to hatching of diagonal lines from an upper right side to a lower left side, a range between value v3 and value v4 is mapped to hatching of dots, a range between value v4 to value v5 is mapped to hatching of a lattice pattern, and a range above value V5 is mapped to unpatterned hatching.

FIG. 8 is an example of mapping a waveform where a change in the index value within one beat is large. As a result, the patterns are frequently changed at a location where the waveform steeply rises, and there is unpatterned hatching which indicates that the value is large. By referring to this, the user can grasp that the change in the index value within the one beat is large.

On the other hand, FIG. 9 is an example of mapping a waveform where a change in the index value within one beat is small. As a result, even at a location where the waveform steeply rises, the change in the patterns is not frequent, and there is no unpatterned hatching which indicates that the value is large. By referring to this, the user can grasp that the change in the index value within the one beat is small.

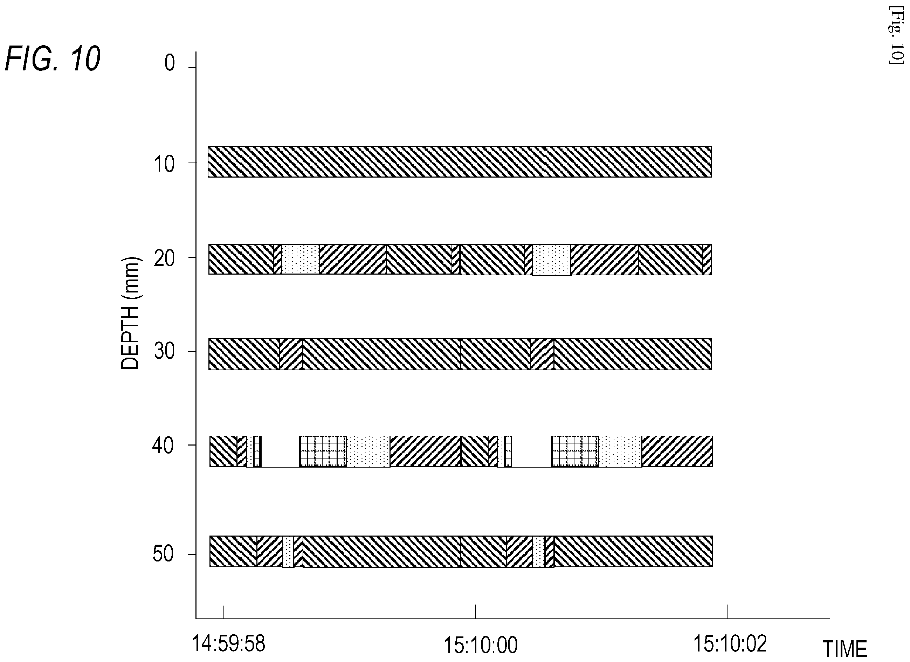

FIG. 10 is an example of the map information created by the control unit 14 through using the concepts of FIGS. 8 and 9. In this example, a bar in which the change in the index value is mapped to the patterns at an interval of 10 mm from a location of 10 mm from the body surface is depicted. In this example, the patterns of the bar frequently change at the position of 40 mm from the body surface, and there is unpatterned hatching which indicates that the value is large. Meanwhile, the patterns of the bar change little at the depth of 10 mm or the depth of 30 mm from the body surface, and there is no unpatterned hatching which indicates that the value is large. Therefore, the user can visually grasp that a motion at the depth of 40 mm from the body surface is large, and a motion at the depth of 10 mm or 30 mm from the body surface is small.

The user may be capable of specifying a time range of a depiction target by operating the operation unit 13 provided on the patient monitor 10 or the remote controller (not illustrated). For example, the user may operate a touch panel (one mode of the operation unit 13) on the patient monitor 10 to specify the time range. The control unit 14 acquires the specified time range (specified time), reads the reflected wave at each depth corresponding to the specified time, and calculates the index value based on the reflected wave. Then the control unit 14 displays a change over time in the calculated index value with a plurality of waveforms and displays the map information in the same manner as in FIGS. 6, 7, and 10. Here, when a time range including a long time (for example, 14: 00 to 16: 00 on February 4, 2020) is specified, the user can grasp "at which time the pulsation is deteriorated", "a speed of the deterioration of the pulsation", and the like.

The user may be capable of explicitly specifying a depth range which serves as a display target. The user specifies the depth range by operating the operation unit 13 (for example, a touch panel, a button, or a knob on the patient monitor 10) or the like. For example, the user specifies a depth of 20 mm to 60 mm, which is considered to be intracranial. Of course, the user may also specify the depth range by a method of specifying a center depth (for example, 30 mm from the body surface) and a depth width (for example, 5 mm for each of a shallow direction and a deep direction). The control unit 14 acquires the specified depth. The control unit 14 acquires the reflected wave at the specified depth from the storage unit 17 or the input and output interface 11, and calculates the index value by the above-described method. Then the control unit 14 displays the change over time in the calculated index value in the same manner as in FIGS. 6, 7, and 10. Since the depth of the display target can be specified in this manner, the user can accurately grasp only the waveform at the depth on which the user wants to focus.

Of course, the user may specify two or more of the time range, the depth range, and the number of waveforms at the same time. The control unit 14 may specify a required reflected wave according to the specification of the user, calculate the index value based on each reflected wave, and then display the waveform.

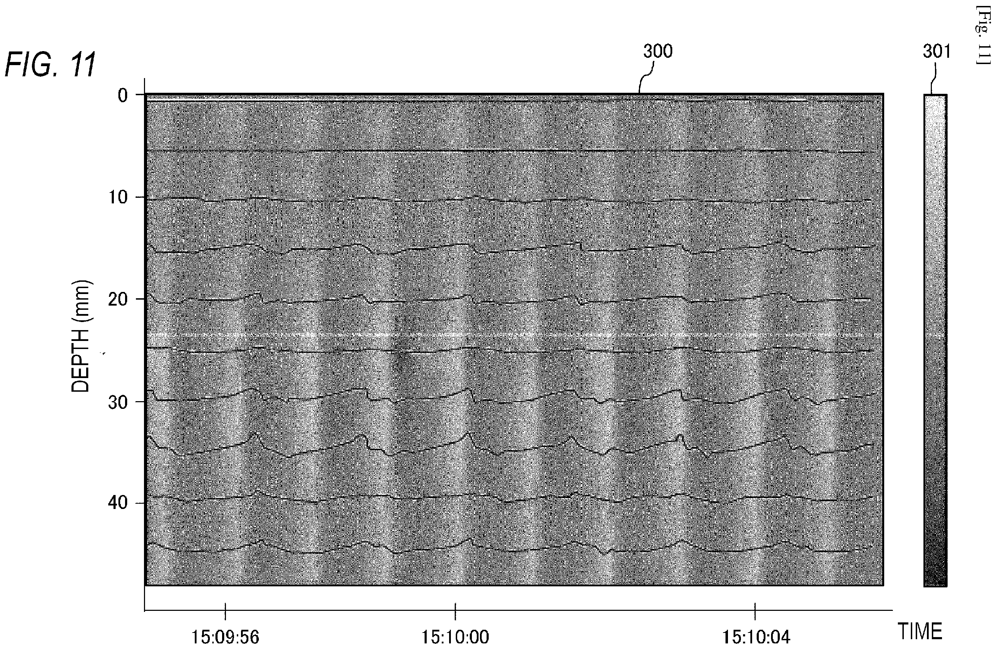

The control unit 14 may also use both of the expressions (A) and (B) ("a plurality of waveforms corresponding to each depth") to display the image data. That is, the control unit 14 may display the image data in which the waveform at each depth is overlapped with the map information indicating the change over time in the index value of each depth. Such an example is illustrated in FIG. 11.

In this example, the control unit 14 continuously maps (to colors) and displays the index value of each depth in the vicinity of 0 mm to 50 mm from the body surface. In addition, the control unit 14 displays a waveform indicating index values of depths at intervals of 5 mm from the body surface of 0 mm in an overlapping manner. In order to facilitate recognition of the waveform, it is desirable that the control unit 14 sets a color of the waveform to a color that is easy to be distinguished from the map information, and performs an adjustment, such as thickening a line of the waveform. The user can comprehensively grasp changes in the pulsation at each depth by referring to such display in which the map information and the plurality of waveforms are displayed in the overlapping manner.

Next, effects of the patient monitor 10 according to the present embodiment will be described. The patient monitor 10 according to the present embodiment acquires the reflected wave indicating the motion generated at each depth by irradiating the ultrasonic wave at the plurality of depths. Then the control unit 14 calculates the index value indicating the motion at each depth from the reflected wave at each depth. The control unit 14 displays the change in the index value of each depth in terms of expression (A) the map information (for example, FIG. 7) expressing a magnitude of the index value on a map taking depth on a first axis and elapsed time on a second axis, by a change in at least one of hue, lightness and chroma. Since the relationship between the depth and the motion is shown by the display, the user can easily grasp the relationship between the depth and the motion at the depth. As a result, the user can easily grasp an abnormal portion, and grasp a difference between the abnormal portion and a normal portion.

A configuration of the basic display control has been described above. Of course, although the example in which the depth increases from an upward direction to a downward direction of the axis of the depth (vertical axis) is shown in the above-described display examples (FIGS. 6, 7, and 10), the depth may also be displayed in such a manner that the depth shallows from the upward direction to the downward direction. Hereinafter, various modifications will be described. Of course, in the following display examples, the depth may shallow from the upward direction to the downward direction.

### <Detection of Change in Pulsation along with Lapse of Time>

When injury occurs on the brain, there may be a case where the pulsation gradually decreases with a lapse of time, or a case where the pulsation increases more than necessary. When a change in the pulsation along with the lapse of time is large, the control unit 14 may detect such a state and notify the state. That is, the control unit 14 uses an index value (for example, the magnitude of the amplitude calculated from the displacement amount) of each depth at a certain time (first time) and an index value (for example, the magnitude of the amplitude calculated from the displacement amount) of each depth at another time (second time) to perform a comparison. Then the control unit 14 performs a notification when it is determined that the change in the index value along with the lapse of time is large based on the comparison. A detailed processing example is as follows.

It is assumed that the control unit 14 displays the change over time in the index value based on the reflected wave at a certain time (first time, preferably a current time) in terms of expression (A). At this time, the control unit 14 reads, from the storage unit 17, the reflected wave at each depth at a time (second time) that is a certain time before the said time (first time). The control unit 14 may also read, from the storage unit 17, a reflected wave to be compared based on any selection criterion as desired instead of the reflected wave before the certain period of time.

As a specific example, the control unit 14 calculates the index value of each depth from the reflected wave at the depth of 20 to 40 mm at the current time (first time). At the same time, the control unit 14 acquires the reflected wave at the depth of 20 to 40 mm one hour before the current time (second time) and calculates the index value of each depth. The control unit 14 may also cache the index value at the certain time before (second time) in the storage unit 17.

Then the control unit 14 uses the index value of the current time and the index value of one hour before to perform the comparison, and determines whether the change in the index value along with the lapse of time is large based on the comparison. Hereinafter, an example of the comparison using the index values will be described. In this example, it is assumed that the index value is the magnitude of the amplitude calculated from the displacement amount. For example, the control unit 14 cuts out one beat from the waveform indicating the displacement amount of 20 mm / 30 mm / 40 mm at the current time, and calculates the magnitude of the amplitude from a difference between a maximum value and a minimum value of the one beat. Of course, an average value or a representative value of a plurality of beats may also be used herein as the magnitude of the amplitude. The control unit 14 calculates the magnitude of the amplitude from the displacement amount of each depth one hour before in the same manner.

The control unit 14 calculates a difference between the magnitude of the amplitude at each depth at the current time and the magnitude of the amplitude at each depth one hour before. For example, the control unit 14 calculates the difference between the magnitude of the amplitude at the depth of 20 mm / 30 mm / 40 mm at the current time and the magnitude of the amplitude at the depth of 20 mm / 30 mm / 40 mm one hour before as follows.
(A) Difference in magnitude of amplitude at 20 mm = magnitude of amplitude at depth of 20 mm at current time - height of amplitude at depth of 20 mm one hour before
(B) Difference in magnitude of amplitude at 30 mm = magnitude of amplitude at depth of 30 mm at current time - magnitude of amplitude at depth of 30 mm one hour before
(C) Difference in magnitude of amplitude at 40 mm = magnitude of amplitude at depth of 40 mm at current time - magnitude of amplitude at depth of 40 mm one hour before

The control unit 14 compares the calculated amplitude difference with a threshold value. For example, the control unit 14 determines whether an average of (A) to (C) described above is equal to or higher than the threshold value. When the amplitude difference is large (equal to or higher than the threshold value), the control unit 14 determines that the change in the pulsation along with the lapse of time is large. When it is determined that the change in the pulsation is large, the control unit 14 notifies that the change in the pulsation is large. The control unit 14 may also compare the calculated amplitude difference with a plurality of threshold values so as to grasp how large the calculated amplitude difference is. For example, the control unit 14 may determine that the change in the index value is "large" when an average of amplitude differences is larger than a threshold value Th1, and may determine that the change in the index value is "very large" when the amplitude difference is larger than a threshold value Th2 (Th1 < Th2).

The control unit 14 may compare the difference of each depth with a threshold value for each depth, and then use a result of the comparison (for example, the number of differences exceeding the threshold value, or a sum of differences between each difference and the threshold value) to determine whether the change in the index value along with the time change is large. For example, the control unit 14 compares the amplitude difference of the depth of 20 mm with a threshold value used for the depth of 20 mm, compares the amplitude difference of the depth of 30 mm with a threshold value used for the depth of 30 mm, and compares the amplitude difference of the depth of 40 mm with a threshold value used for the depth of 40 mm. Then the control unit 14 may determine whether the change in the index value along with the time change is large based on a result of each comparison (for example, whether the number of differences exceeding the threshold values is two or more).

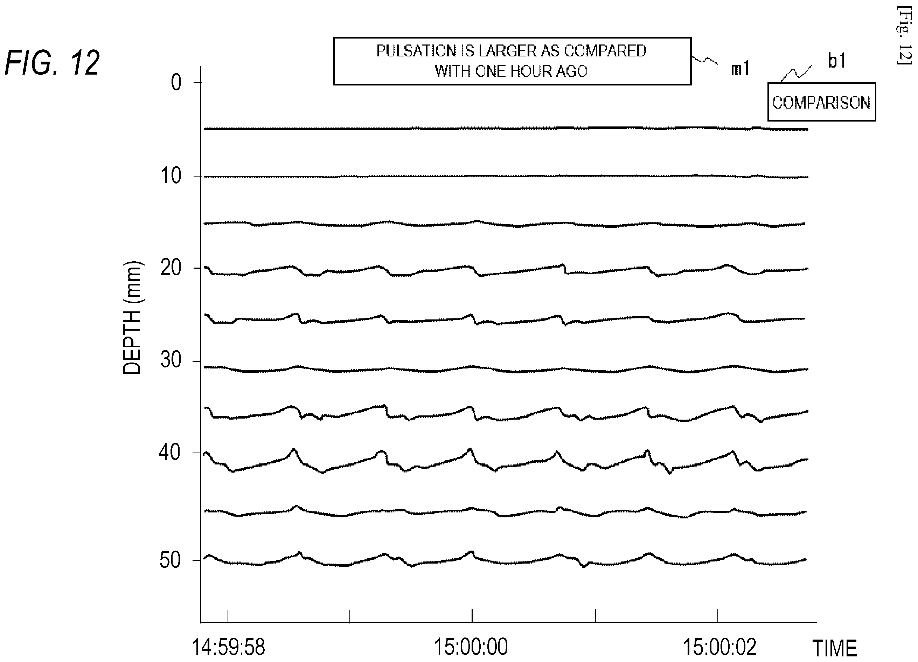

When it is determined that the change in the index value is large, the control unit 14 performs the notification by sound or display. Examples of methods of the notification will be listed with reference to FIGS. 12 to 14. For example, when it is detected that the change in the index value is large, the control unit 14 may output an alarm sound via the speaker 15. Here, a type of the alarm sound (such as tones indicating a degree of urgency) and volume may be changed according to magnitude of the change (for example, magnitude of a value exceeding the threshold value). For example, the control unit 14 sounds an emergency alarm when it is determined that the change in the pulsation is very large, and sounds a normal alarm when it is determined that the change in the pulsation is large.

The control unit 14 may also display a message alerting that the change in the index value is large in terms of at least one of the map information ((A) described above) and "a plurality of waveforms corresponding to each depth" . For example, as illustrated in FIG. 12, the control unit 14 may display a message m1 "pulsation is larger as compared with one hour ago" on a display screen of each waveform at the plurality of depths. Further, the control unit 14 may display, at the same time as the message m1, an interface (in this example, a "comparison" button b1) through which a screen display capable of being compared with a past change over time in the index value can be specified.

### <Display of Comparison along with Lapse of Time>

When the button b1 is operated, the control unit 14 may display a past change over time in the index value (in terms of at least one of the map information ((A) described above) and "a plurality of waveforms corresponding to each depth" and a current change over time in the index value (in terms of at least one of the map information ((A) described above) and "a plurality of waveforms corresponding to each depth" together. The control unit 14 may use a past reflected wave or calculated index value to display past pulsation in the same manner as the method described above.

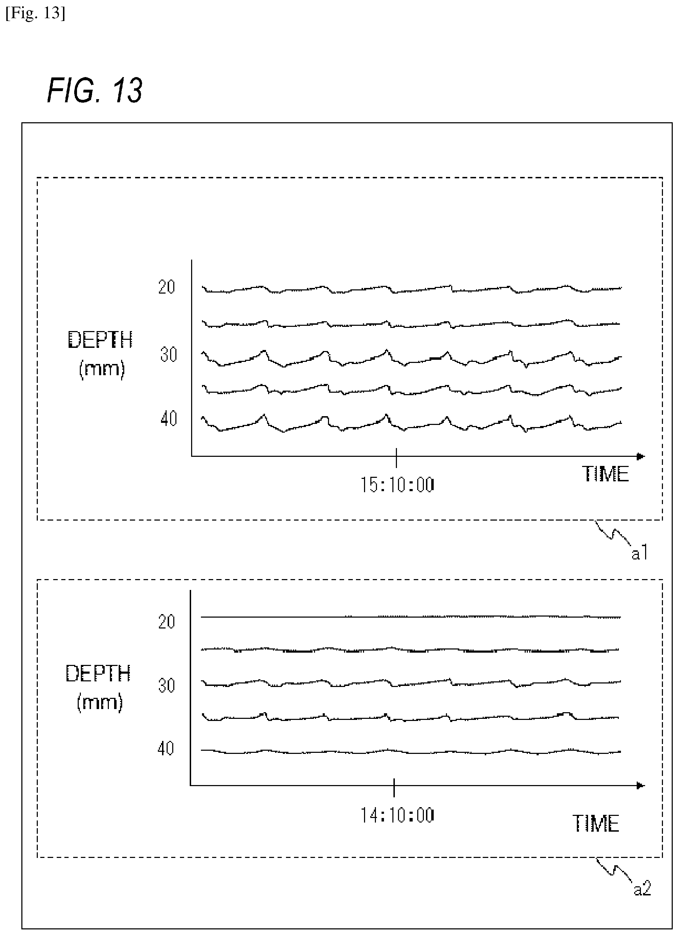

For example, as illustrated in FIG. 13, the control unit 14 displays an area a2 where a plurality of past waveforms (near 14: 00: 00) are displayed and an area a1 where a plurality of current waveforms (15: 00: 00) are displayed. Although the display is vertically arranged in this example, the display may also be horizontally arranged. Even when the control unit 14 displays the map information, the control unit 14 may display a plurality of pieces of map information in parallel on left, right, upper, and lower sides.

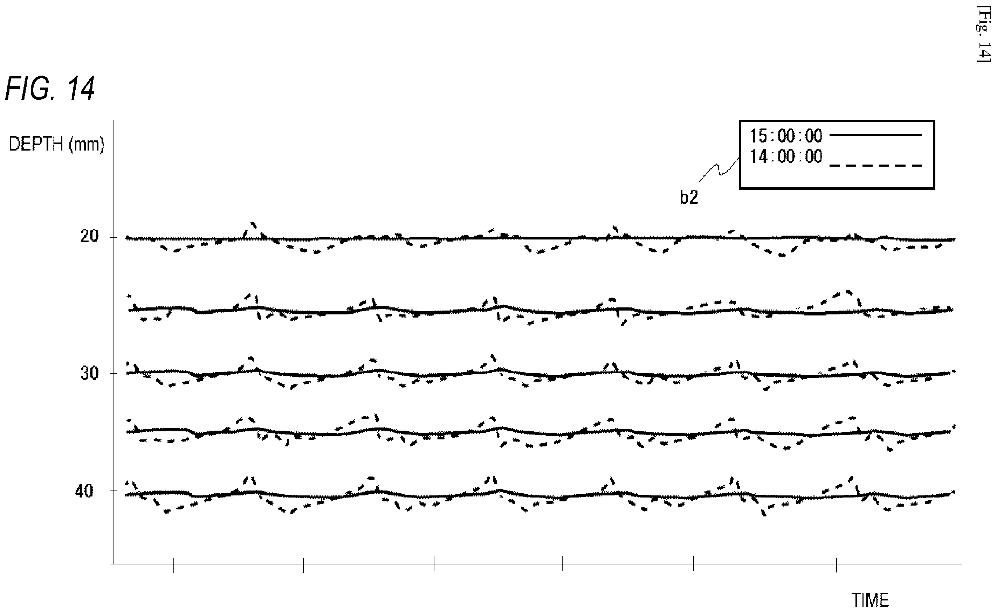

As illustrated in FIG. 14, the control unit 14 may display the plurality of past waveforms (near 14: 00: 00) and the plurality of current waveforms (15: 00: 00) in an overlapping manner at the same position. In FIG. 14, the control unit 14 displays the waveforms at 14: 00: 00 and the waveforms at 15: 00: 00 in the overlapping manner after aligning display positions of the respective depths. As illustrated in the drawing, it is preferable that the control unit 14 changes a line type, thickness, color, end point pattern, and the like of the waveform in accordance with a difference in time zones, and in this example, the line type (solid line and dotted line) is changed. Further, it is more preferable that the control unit 14 displays an explanatory note B2 that indicates the difference in time zones.

When the control unit 14 performs the above-described comparison and determines that the change in the index value is larger than the threshold value (the change is larger as compared with past pulsation), the control unit 14 may automatically switch to a display (FIGS. 13 and 14) through which the past index value and the current index value can be compared with each other. For example, when the control unit 14 determines that the change in the index value is larger than the threshold value (the change is larger as compared with the past pulsation), the control unit 14 may automatically switch from waveform display illustrated in FIG. 6 to parallel waveform display illustrated in FIG. 13.

As described above, the control unit 14 compares the index value at the certain time with the index value at another time, and performs the notification when the change in the index value is large. Accordingly, the user can clearly grasp the fact that the change in the index value with the lapse of time is large. When grasping such a fact, the user can visually and easily grasp how much change has occurred by the performed display through which the plurality of waveforms or map information can be compared.

Even when it is determined that the change in the index value along with the lapse of time is small, the control unit 14 may constantly display the operation interface (for example, the comparison button b1 illustrated in FIG. 12) used for displaying the past waveforms at the plurality of depths or the map information. That is, the control unit 14 may constantly display the "comparison" button b1 illustrated in FIG. 12 on the display screen of the waveforms at the plurality of depths as illustrated in FIG. 6, for example. The control unit 14 may also perform display through which the current plurality of waveforms / map information and the past plurality of waveforms / map information can be constantly compared according to setting (selection of a "constant comparison mode") of the user without performing the above-described comparison process.

That is, the control unit 14 may display both the change over time in the index value of each depth at the first time and the change over time in the index value of each depth at the second time not only when an abnormality is detected along with the lapse of time but also when the user performs the operation or setting. For example, the control unit 14 may display the two waveform areas as illustrated in FIG. 13 or may display two pieces of map information in response to a button operation performed by the user. The user may specify not only the index value of the current time but also two or more time zones to be compared (for example, a comparison between one hour ago and two hours ago), and the control unit 14 may perform display (parallel display, overlapped display, or the like) through which changes over time in index values of the specified time zones can be compared.

Although it is preferable that the control unit 14 displays the two waveform areas (or map information) side by side (for example, as illustrated in FIG. 13, X coordinates of start points and X coordinates of end points of time axes of two graphs coincide with each other), the two waveform areas may be displayed at any position as desired. For example, the control unit 14 may be configured to freely move the waveform areas in accordance with a mouse operation performed by the user. In other words, the control unit 14 may display the two waveform areas (or map information) side by side in an oblique direction instead of in a vertical direction or a horizontal direction, or may display in such a manner that parts of the two waveform areas overlap each other.

The control unit 14 may also constantly perform the overlapped display illustrated in FIG. 14. At this time, it is desirable that the control unit 14 differentiates a display mode (for example, a thickness of a line, a line type, a color, or a pattern of an end point) of the waveform indicating the first time from a display mode of the waveform indicating the second time.

### <Alignment of Feature Points>

The control unit 14 may perform control to arrange an edge timing, which is a type of feature point of a waveform group at the first time (first waveform group), and an edge timing, which is a type of feature point of a waveform group at the second time (second waveform group) at corresponding positions so as to display the waveform group at the first time and the waveform group at the second time. A concept of such display control will be described below.

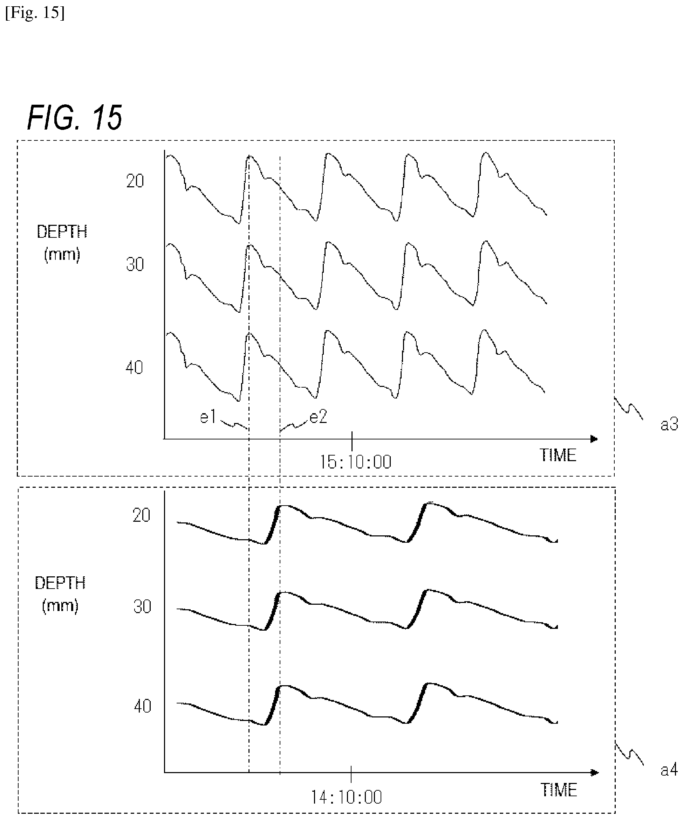

As illustrated in FIG. 2 and the like, since the pulsation waveform is generated due to beats of the heart, the pulsation waveform is a so-called pulsation waveform (a waveform in which peak points are periodically generated). Therefore, when positions of peak points of two waveforms are aligned and displayed, it is easy for the user to accurately grasp a difference between shapes of the two waveforms (the magnitude of the amplitude and the like). For example, FIG. 15 illustrates an example in which a plurality of waveforms at a certain time point (15: 10: 00) and a plurality of waveforms one hour before the certain time point (14: 10: 00) are simply arranged in the vertical direction. In other words, this is an example in which an X coordinate at 15: 10: 00 and an X coordinate at 14: 10: 00 are the same. As illustrated in the drawing, there is a deviation in a horizontal axis direction at a timing when the waveform rises / falls greatly (hereinafter also referred to as the edge timing). Specifically, there is a deviation between horizontal axis positions of an edge timing e1 of a waveform area a3 and an edge timing e2 of the waveform area a4. Such a deviation is mainly caused by speed of a heartbeat. If there is such a deviation, a user may feel difficult to compare waveform amplitudes.

Since the index value of the change over time is caused by the heart pulsation, it is general that there is no deviation (or the deviation is very small) at the edge timing even if depths are different at the same time. In the following description, it is assumed that there is no deviation at the edge timing at each depth at the same time.

The control unit 14 may perform a process of aligning the edge timings of the waveform groups at two specified times and then perform display. For example, the control unit 14 detects the edge timing of the waveform group indicating the change over time in the index value at the first time / the second time by the same method as detection of an R wave of a general electrocardiogram waveform. Then the control unit 14 performs display in such a manner that the edge timing of the waveform group indicating the change over time in the index value at the first time and the edge timing of the waveform group indicating the change over time in the index value at the second time are arranged at corresponding positions.

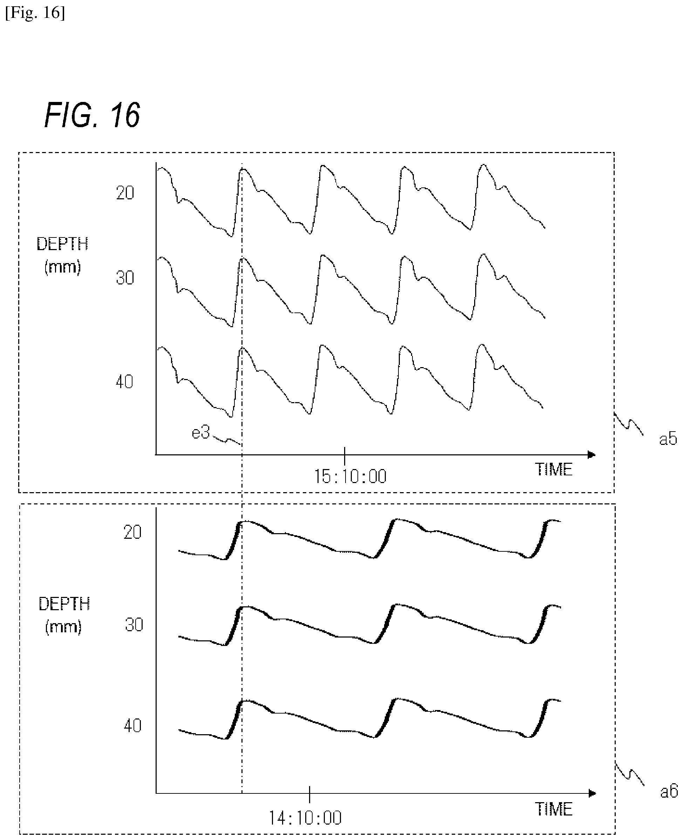

FIG. 16 illustrates such an example. The control unit 14 detects the edge timing of the waveform group at the first time and detects the edge timing of the waveform group at the second time. Then the control unit 14 displays the two waveform areas a5 and a6 in such a manner that positions of the detected edge timings are located at the corresponding positions. For example, the control unit 14 sets horizontal axis positions (X coordinates) of the edge timings to be the same, and displays a waveform group around a predetermined time from the edge timing (for example, from one second ago to four seconds thereafter). As a result, a position (X coordinate) of the edge timing e3 is aligned, and then the waveform area a5 and the waveform area a6 are displayed. Although the waveform area a6 is intended to display the waveform group one hour before the waveform area a5, since the position of the edge timing is aligned, a time thereof is not strictly one hour before (in other words, there is a deviation between a horizontal axis position at 15: 10: 00 and a horizontal axis position at 14: 10: 00).

Of course, the control unit 14 may also clearly indicate the edge timing e3 which is detected to align a display position as illustrated in FIG. 16. That is, the control unit 14 may display information (in the example of FIG. 16, a one-dot chain line indicating the edge timing e3) indicating the edge timing (feature point).

Although the control unit 14 detects the edge timing of the waveform group at the first time and the edge timing of the waveform group at the second time, the presently disclosed subject matter is not necessarily limited thereto. The edge timing (peak point) is an example of the feature point of the waveform, and for example, any minimum value / maximum value / inflection point of the waveform group may be used as the feature point. In a case where the control unit 14 detects any minimum value or the like as the feature point, the same process as the display control using the R wave described above may still be performed.

### <Divided Display of Beat>

The control unit 14 may perform processing to facilitate a comparison of a difference between the two waveform groups for each beat. For example, the control unit 14 divides (cuts) the waveform group indicating the change over time in the index value at the first time (first waveform group) into beats, and divides the waveform group indicating the change over time in the index value at the second time (second waveform group) into beats. Then the control unit 14 compares a beat time interval of the waveform group at the first time with a beat time interval of the waveform group at the second time. Based on the comparison, the control unit 14 detects a long period waveform group whose time interval is longer than the other waveform group and a short period waveform group whose time interval is shorter than the other waveform group. Then the control unit 14 displays both of the waveform groups together in such a manner that one beat of the short period waveform group is included in the time interval of one beat of the long period waveform group.

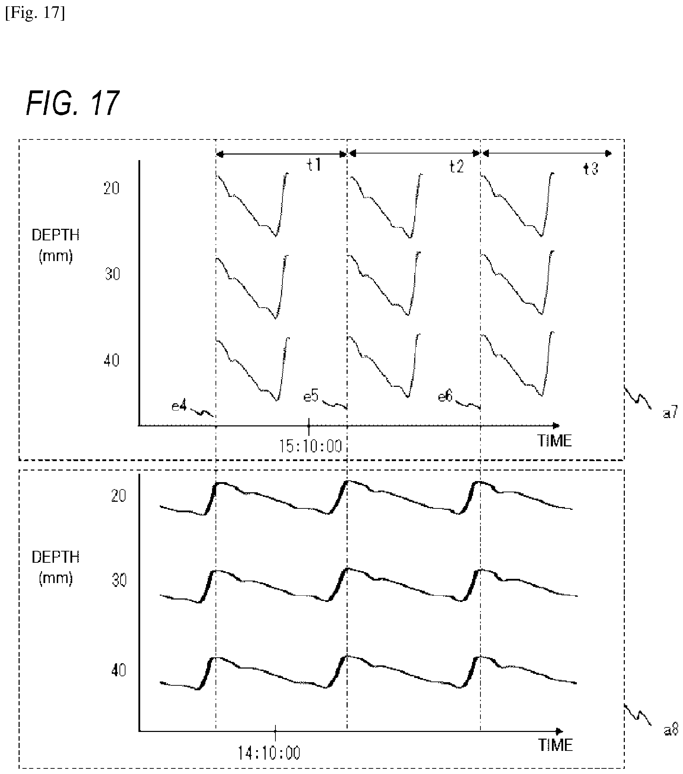

FIG. 17 is an example of such display. The control unit 14 divides a waveform group of a waveform area a7 into beats, and divides a waveform group of a waveform area a8 into beats. Then the control unit 14 compares a time interval between the beats divided from the waveform group of the waveform area a7 with a time interval between the beats divided from the waveform group of the waveform area a8. The control unit 14 determines that the beat time interval of the waveform area a8 is long. That is, the control unit 14 detects the waveform group of the waveform area a8 as the long period waveform group and detects the waveform group of the waveform area a7 as the short period waveform group. The control unit 14 displays the two waveform areas a7 and a8 together in such a manner that one beat divided from the short period waveform group a7 is included in a time interval t1 / t2 / t3 of one beat of the long period waveform group a8. As a result, when there is a difference between the beat time intervals, the waveform of the waveform area a7, whose beat time interval is shorter, is intermittent as illustrated in FIG. 17. By referring to such display, the user can more clearly grasp a difference in a length of one beat or a difference in the height of the amplitude along with the lapse of time.

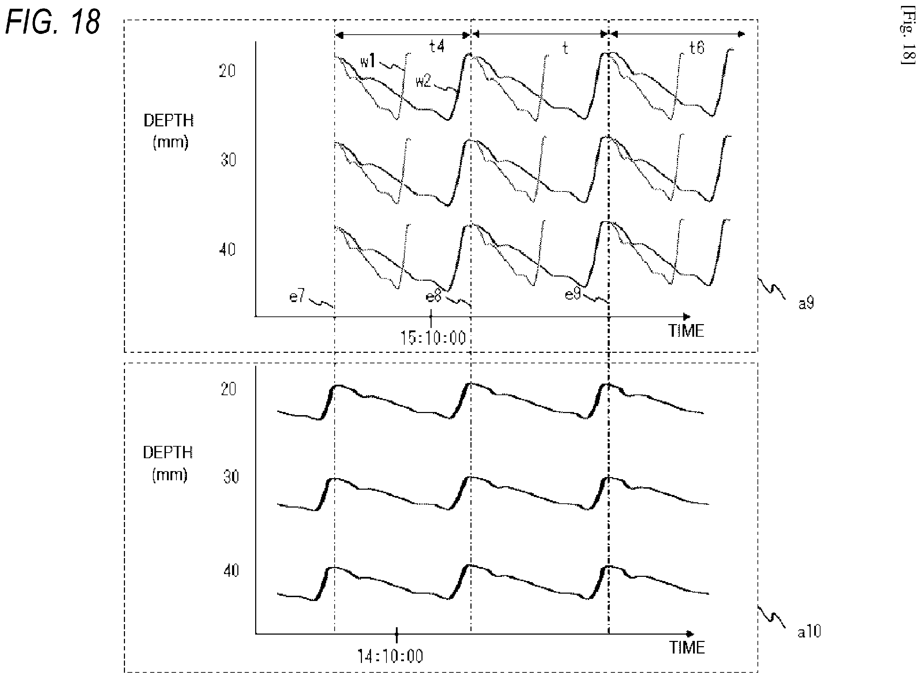

The control unit 14 may also extend and display the beats of the short period waveform group in accordance with the beat time interval of the long period waveform group. FIG. 18 illustrates an example. The control unit 14 divides the beats from the waveform group, compares the time intervals between the divided beats, and displays the beats in the same manner as in FIG. 17. In addition, the control unit 14 also displays a waveform of a beat obtained by extending a beat of a waveform area a9 determined to have a short beat time interval (short period waveform group) in accordance with time intervals t4, t5, and t6 of a waveform area a10 whose beat time interval is long (long period waveform group). For example, the control unit 14 displays an extended waveform w2 in addition to a waveform w1 divided in the waveform area a9. Of course, the control unit 14 may only display the extended waveform in the waveform area a9 (that is, only the waveform corresponding to the waveform w2). Such display may be useful when it is desired to compare the amplitudes of the waveforms along with the lapse of time.

Although the edge timing (falling timing of the waveform) is used as a beat division point in the display examples of FIGS. 17 and 18, the presently disclosed subject matter is not necessarily limited thereto. For example, when a length of one beat is about 1 to 1.5 seconds, the control unit 14 divides the waveform into beats with 0.3 to 0.5 seconds before the falling timing of the waveform serving as a start point of the beat. Such division facilitates comparison between falling timings of beats when the beats are displayed.

Concepts of the waveform display illustrated in FIGS. 16 to 18 can also be applied when waveforms in different time zones are displayed in the overlapped manner as illustrated in FIG. 14. For example, the control unit 14 detects the edge timing from both the waveform group indicating the change over time in the index value at the first time and the waveform group indicating the change over time in the index value at the second time in the same manner as described above. Then the control unit 14 displays the waveform at the first time and the waveform at the second time in an overlapping manner such that the positions of the edge timings are the same. For example, when a waveform at 14: 00: 00 and a waveform at 15: 00: 00 are displayed in the overlapped manner, the control unit 14 may display waveform groups at each time in the overlapped manner by setting a position (X coordinate) of an edge timing closest to 14: 00: 00 and a position (X coordinate) of an edge timing closest to 15: 00: 00 to the same position (the same X coordinate value).

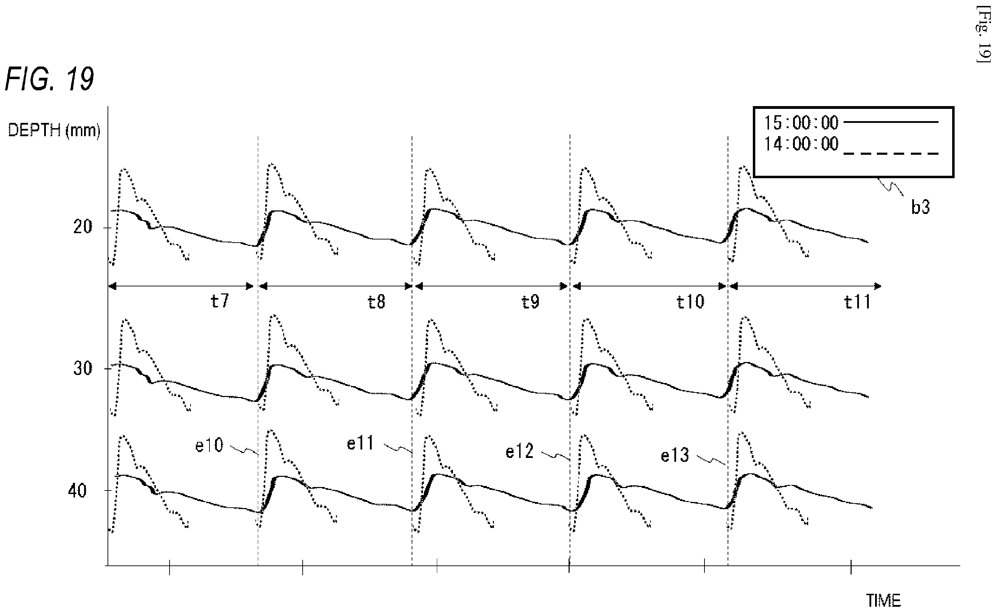

The control unit 14 may also perform the divided display of beats corresponding to FIG. 17 when waveform groups at two times are displayed in the overlapped manner. FIG. 19 is an example of such display. In the example of FIG. 19, the control unit 14 divides a waveform group measured near 14: 00: 00 into beats, and divides a waveform group measured near 15: 00: 00 into beats. Then the control unit 14 compares lengths of beat time intervals, and determines that the beat time interval of the waveform group measured near 15: 00: 00 is longer. That is, the control unit 14 detects the waveform group measured near 15: 00: 00 as the long period waveform group and detects the waveform measured near 14: 00: 00 as the short period waveform group. The control unit 14 displays the long period waveform group and the short period waveform group in the overlapped manner such that one beat divided from the short period waveform group (the waveform measured near 14: 00: 00) is included in time intervals t7 to t11 of one beat of the long period waveform group (the waveform measured near 15: 00: 00).

The control unit 14 may also explicitly indicate edge timings e9 to e13 as illustrated in FIG. 19, or may display an explanatory note b3 which indicates a type of the waveform. Although not illustrated, the control unit 14 may also perform processing of extending the waveform in a time axis direction even when the waveform groups at two times are displayed in the overlapped manner as in the method illustrated in FIG. 18.

### <Detection of Change in Pulsation along with Depth Change>

When injury occurs on the brain or the like, there may be a case where a degree of damage varies from place to place in the brain depending on a location where a force is applied at the time of the injury and magnitude of the force. For example, there may be a case where pulsation at some depths is extremely large or small depending on the location where pressure is applied due to the injury or magnitude of the pressure. The control unit 14 compares index values of the respective depths at the same time to determine whether there is any depth having a large difference in the index value as compared with surroundings. Then the control unit 14 may perform the notification (alarm sounding, message display, or the like) in a case where there is a depth having an abnormal index value as compared with the surroundings. A detailed processing example will be described below.

For example, before displaying at least one of the map information ((A) described above) and "a plurality of waveforms corresponding to each depth", the control unit 14 uses the index value of each depth to perform the comparison. Hereinafter, it is assumed that the index value is the magnitude of the amplitude calculated from the displacement amount. For example, the control unit 14 sets one beat from a peak point to a next peak point of the displacement amount at the depth of 0 mm, and calculates the magnitude of the amplitude within the one beat (difference between a maximum value and a minimum value within the one beat). The control unit 14 calculates the magnitude of the amplitude within one beat at the depth of 10 mm (the difference between the maximum value and the minimum value within the one beat) in the same manner. Then the control unit 14 compares a difference between the magnitude of the amplitude at the depth of 0 mm and the magnitude of the amplitude at the depth of 10 mm with a predetermined threshold value, and determines that the change in the index value depending on the change in the depth is large when the difference is larger than the threshold value. Based on such determination, the control unit 14 specifies a location where the change in the index value depending on the depth is large.

The control unit 14 performs a comparison between the depth of 10 mm and the depth of 20 mm, a comparison between the depth of 20 mm and the depth of 30 mm, and the like by the same method. Then the control unit 14 specifies a location where the difference in the index value depending on the depth is large. For example, the control unit 14 determines that a location at the depth of 30 mm is a location where the change in the index value is larger as compared with surrounding locations.

Here, when determining whether the change in the index value of a certain specific depth (first depth) is large, it is preferable that the control unit 14 uses an index value of the certain depth and an index value of a location (second depth) in the shallow direction from the certain depth to perform a comparison, and uses the index value of the certain location (first depth) and an index value of a location (third depth) in the deep direction from the certain location to perform a comparison. As a result, the control unit 14 determines whether the depth is a depth where index value has a large difference from the surroundings. For example, the control unit 14 compares a difference between the magnitude of the amplitude at the depth of 30 mm and the magnitude of the amplitude at the depth of 20 mm with the threshold value, and compares a difference between the magnitude of the amplitude at the depth of 30 mm and the magnitude of the amplitude at the depth of 40 mm with the threshold value. Then the control unit 14 may determine that the amplitude at the depth of 30 mm is abnormal as compared with peripheral locations when it is determined that the difference between the amplitudes is larger than the threshold value through the comparison between the two depths. The control unit 14 may also determine whether the amplitude at the depth of 30 mm is abnormal by comparing a sum of the difference between the amplitude at the depth of 30 mm and the amplitude at the depth of 20 mm and the difference between the amplitude at the depth of 30 mm and the amplitude at the depth of 40 mm with the threshold value. Of course, the control unit 14 may also compare the index value of the certain depth with three or more depths when it is determined that the index value of the certain depth is abnormal as compared with the surroundings. By comparing with both of the position shallower than the certain depth and the position deeper than the certain depth during the determination of the abnormality of the index value of the certain depth, it is possible to accurately specify a local abnormal location.

In general, the pulsation caused by the heartbeat is very small on an outer side of the cranial bone or in the vicinity of the cranial bone due to an influence of bone strength. Therefore, the control unit 14 may exclude the amplitude from the body surface to a predetermined depth (for example, 15 mm) from comparison targets.

The control unit 14 may determine that an index value of a certain depth is abnormal when a state where a difference between the index value of the certain depth and an index value of a peripheral depth is continuously large for a certain or longer period of time. The control unit 14 may also detect a level of abnormality of the amplitude at the certain depth (for example, level 1: slightly abnormal, level 2: abnormal, and level 3: significantly abnormal) by comparing a plurality of threshold values with the difference between the amplitudes.

When the control unit 14 detects the location where the index value is abnormal as compared with other depths, the control unit 14 notifies that the index value is abnormal at the certain depth by sound or display. For example, the control unit 14 outputs an alarm sound via the speaker 15. Here, a type of the alarm sound (such as tones indicating the degree of urgency) and volume may be changed according to a magnitude level of the difference between the certain depth and another depth (for example, magnitude of a value exceeding the threshold value).

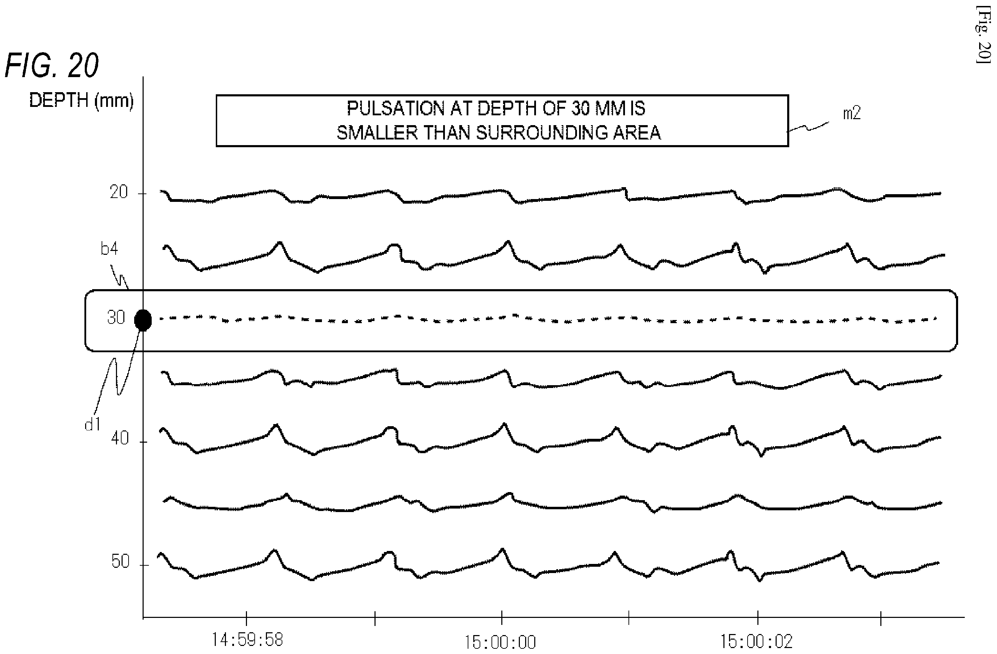

The control unit 14 may also display a message alerting that there is an abnormality of the index value at the certain depth at the time of displaying at least one of the map information ((A) described above) and the "a plurality of waveforms corresponding to each depth"). Hereinafter, a display example will be described with reference to FIG. 20.

In FIG. 20, it is assumed that the depth of 20 mm to 50 mm is specified as the display target. As illustrated in FIG. 20, the control unit 14 may display a message m2 "pulsation at depth of 30 mm is smaller than surrounding area" on the display screen of each waveform at the plurality of depths. Further, in order to notify the abnormal depth, the control unit 14 may differentiate the line type, color, thickness, and end point pattern of the waveform of the depth from those of waveforms of other depths. In the example of FIG. 20, a waveform at the depth of 30 mm where the pulsation is abnormal is indicated by a dotted line, and waveforms at other depths are indicated by solid lines. Also as illustrated in FIG. 20, a dot d1 may be displayed at a vertical axis position of the depth where the abnormal pulsation occurs, a position of the depth of 30 mm may be surrounded by a solid line b4 and displayed in a manner distinguishable from other depths, or a background color near the depth may be changed. That is, it is preferable that the control unit 14 displays in such a manner that the depth where the index value is abnormal can be distinguished as compared with peripheral depths.

The control unit 14 may still perform the same processing at the time of displaying the map information so as to display in such a manner that the depth where the index value is abnormal can be distinguished as compared with the peripheral depths. For example, the control unit 14 may display the dot at the vertical axis position of the depth where the index value is abnormal, or may display in such a manner that a display location of the depth where the index value is abnormal is surrounded with a dotted line or the like so as to be distinguishable from other depths. The control unit 14 may also notify the depth where the index value is abnormal by displaying a message (for example, "pulsation at depth of 30 mm is smaller than surrounding area").

### <Viewpoint of Two-Point Comparison>

When a depth to be compared with a depth to be used as a reference is clear, the patient monitor 10 can be interpreted as a patient monitor that performs an operation of displaying the map information and the plurality of waveforms described above for the two depths.

The ultrasonic wave transducer 100 irradiates the ultrasonic wave at the first depth which serves as the reference so as to acquire the reflected wave, associates the reflected wave (first reflected wave) with the first depth, and supplies the associated reflected wave and first depth to the input and output interface 11. The ultrasonic wave transducer 100 also irradiates the ultrasonic wave at the second depth which serves as a comparison target so as to acquire the reflected wave, associates the reflected wave (second reflected waves) with the second depth, and supplies the associated reflected wave and second depth to the input and output interface 11.

The input and output interface 11 acquires the first reflected wave related to a motion at the first depth and the second reflected wave related to the second depth. The control unit 14 calculates an index value (first index value), such as the displacement amount, by the above-described method using the first reflected wave, and calculates an index value (second index value), such as the displacement amount, by the above-described method using the second reflected wave.

The control unit 14 displays changes over time in the first index value and the second index value in terms of expression (A) in the same manner as described above. As a result, the user can explicitly grasp a difference in pulsation of the comparison target as compared with pulsation of the reference.

When the waveforms are displayed, the control unit 14 may display the waveforms in parallel in such a manner that the waveforms do not overlap each other as in FIG. 6 and the like, or may display the waveforms at the same height position in the overlapped manner such that depths thereof are distinguishable. For example, the control unit 14 may display the waveform of the first depth in red and the waveform of the second depth in blue in the overlapped manner at the same height, and then display an explanatory note related to the display of the waveforms together (for example, a label indicating that a red line waveform is at the first depth while a blue line waveform is at the second depth). By displaying the two waveforms at the same height in the overlapped manner, the user can more clearly grasp what the pulsation of the comparison target is like as compared with the pulsation of the reference (how large or small the amplitude is, what is the shape of the waveform, and the like).

Of course, a configuration in which there is a plurality of comparison targets (for example, a configuration in which a third depth is also set as the comparison target) may be employed.

### <Modification of Physiological Information Processing System 1>

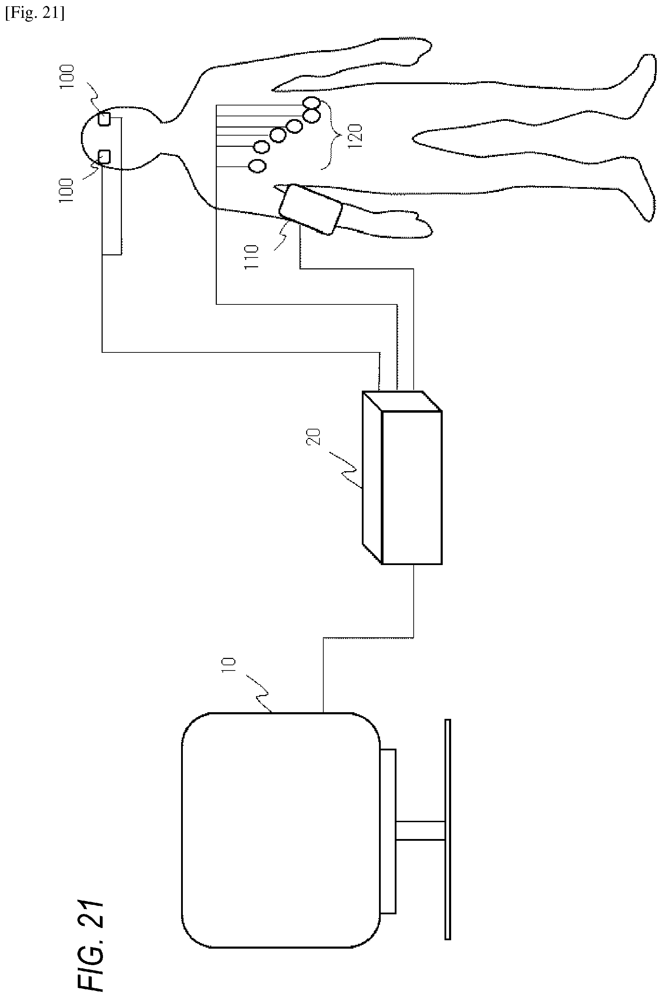

The physiological information processing system 1 may also be configured to acquire a physiological signal via an input unit. Such a modification is illustrated in FIG. 21. The physiological information processing system 1 includes the patient monitor 10 and an input unit 20. The input unit 20 relays various signals between each sensor (the ultrasonic wave transducer 100, the cuff 110, and the electrocardiogram electrode 120) and the patient monitor 10.

The input unit 20 may be configured to perform a part or all of signal processing performed by the patient monitor and processing such as generation of a display image. That is, the input unit 20 may be configured to perform display control or the like based on the reflected wave acquired from the ultrasonic wave transducer 100 described above, and transmit display image data to the patient monitor 10. In this case, the input unit 20 operates as an aspect of the patient monitor including the control unit 14 that performs the above-described processing, the input and output interface 11, and the like.

### <Application to Display of Other Vital Sign Waveforms>

The display concepts of FIGS. 16 to 19 are applicable not only to the case of displaying the waveforms of the index values corresponding to the plurality of depths, but also to a case of displaying a waveform of a general vital sign (for example, a blood pressure waveform, an electrocardiogram waveform, or a respiration waveform). That is, the presently disclosed subject matter can also be applied to a case where a waveform (first physiological waveform) of a vital sign at a first time and a waveform (second physiological information) of a vital sign of the same type at a second time are displayed together. Details thereof will be described below.

As illustrated in FIG. 3, the patient monitor 10 can be connected to the cuff 110 and the electrocardiogram electrode 120. Of course, the patient monitor 10 may also be connected to a respiration sensor, a respiration mask, an SpO2 probe, or the like (not illustrated) which can acquire a respiration waveform.

The input and output interface 11 acquires the physiological signal from the various sensors (such as the cuff 110 and the electrocardiogram electrode 120). Although measurement of electrocardiogram or respiration depends on a medical condition of the subject, it is assumed that the measurement is performed continuously for a certain long time (for example, 30 minutes or more). That is, the input and output interface 11 acquires the physiological signal at the first time and the physiological signal at the second time by continuous measurement.

The input and output interface 11 supplies the physiological signal acquired from the various sensors to the control unit 14. The control unit 14 performs various types of processing (for example, digitalization processing and filtering processing) on the acquired physiological signal, and detects a physiological waveform and a measurement value of each vital sign. In this example, it is assumed that the control unit 14 detects an electrocardiogram waveform based on an electrocardiogram signal acquired from the electrocardiogram electrode 120. Detection of the electrocardiogram waveform may be performed by the same method as that of a general patient monitor. The control unit 14 writes data in the storage unit 17 as appropriate for long-term storage of the data, and reads data necessary for display or the like from the storage unit 17 as appropriate.

The control unit 14 displays the physiological waveform and the measurement value in real time in the same manner as the general patient monitor. When the user operates the operation unit 13 to select a mode of comparing waveforms in two time zones, the following operation is performed. The user may also explicitly specify a time zone (first time, second time) to serve as the comparison target. In the following description, it is assumed that it is specified to compare a current (first time) electrocardiogram waveform with an electrocardiogram waveform one hour before (second time).

The control unit 14 detects a physiological waveform at the first time and a physiological waveform at the second time based on the physiological signal acquired from the various sensors. The physiological waveform at the first time and the physiological waveform at the second time are based on the same physiological signal, and are physiological waveforms of the same type (electrocardiogram waveforms in this example).

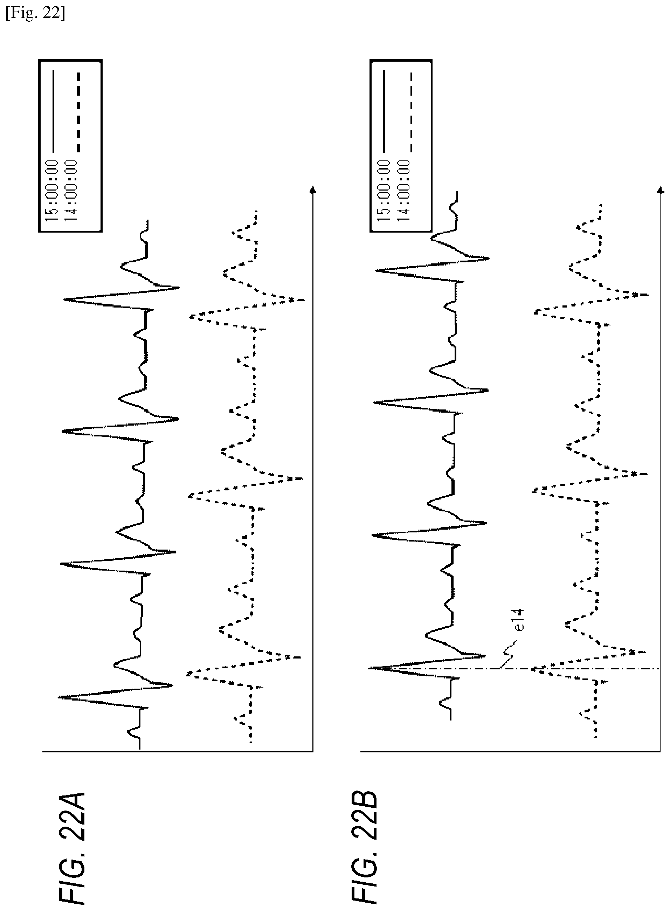

The control unit 14 analyzes the electrocardiogram waveform at the current time (first physiological waveform) and detects the feature point of the electrocardiogram waveform. In this example, it is assumed that the feature point is an R wave. The control unit 14 analyzes the electrocardiogram waveform one hour before (second physiological waveform) and detects an R wave of the electrocardiogram waveform in the same manner. Then the control unit 14 displays the current electrocardiogram waveform and the electrocardiogram waveform one hour before in such a manner that timings of the R waves are arranged at corresponding positions. FIGS. 22A and 22B are an example of such display.

FIG. 22A is a reference diagram in which the current electrocardiogram waveform and the electrocardiogram waveform one hour before are vertically arranged. On the other hand, FIG. 22B is a display example generated by the control unit 14, and is an example in which the R wave timings of the current electrocardiogram waveform and the electrocardiogram waveform one hour before are aligned to be located in the same position on a time axis and displayed. The control unit 14 arranges the timings of the detected R waves at the corresponding positions (in this example, positions at which X-axis coordinates have the same value), and displays the current electrocardiogram waveform and the electrocardiogram waveform one hour before (FIG. 22B). The control unit 14 also displays information on the corresponding positions together (one-dot chain line e14 indicating the timing of the R wave). In this way, when the two physiological waveforms of the same type in different time zones (the first time and the second time) are displayed together, the user can accurately grasp a difference between shapes of the waveforms by arranging the feature points (for example, the timings of the R waves) of the two waveforms at the corresponding positions and displaying the two types of waveforms (FIG. 22B).

The feature point may also be an S wave or a T wave instead of the R wave, and may be a timing before a predetermined time (or after the predetermined time) from the R wave. That is, the feature point is a concept indicating a point that can be extracted by a waveform analysis.

In the example of FIG. 22B, the R waves (one type of feature point) of the two waveforms are arranged at the corresponding positions (with the same X coordinate) and the two types of waveforms are displayed side by side. However, of course, the R waves (one type of feature point) of the two waveforms may also be arranged at the corresponding positions (with the same X coordinate) and then displayed in an overlapped manner.

The control unit 14 may perform the display after performing the processing that facilitates the comparison of the difference for each beat. The control unit 14 divides the current electrocardiogram waveform into beats and divides the electrocardiogram waveform one hour before into beats. The control unit 14 compares a beat time interval of the current electrocardiogram waveform with a beat time interval of the past electrocardiogram waveform. Based on the comparison, the control unit 14 detects a long period waveform whose time interval is longer than the other waveform and a short period waveform whose time interval is shorter than the other waveform. Then the control unit 14 displays both of the waveforms together in such a manner that one beat of the short period waveform is included in the time interval of one beat of the long period waveform.

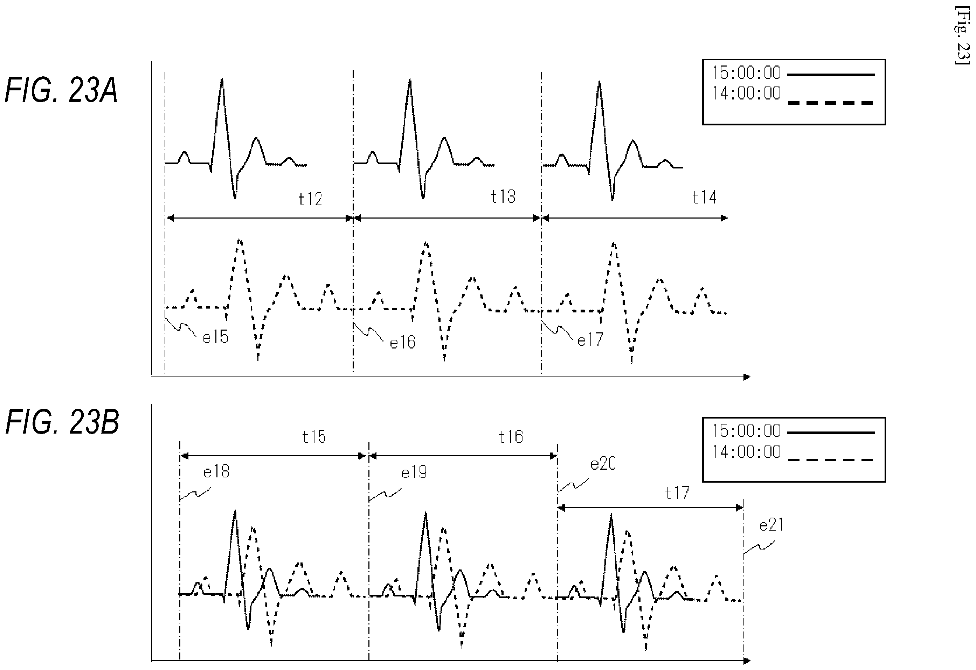

FIG. 23A is an example of such display. The control unit 14 divides the current electrocardiogram waveform (near 15: 00: 00) into beats, and divides the electrocardiogram waveform one hour before (near 14: 00: 00) into beats. The beat division may be performed through using any method used in a general electrocardiogram analysis. Then the control unit 14 compares the time interval between the beats divided from the current electrocardiogram waveform with the time interval between the beats divided from the electrocardiogram waveform one hour before. In this example, the control unit 14 detects the electrocardiogram waveform one hour before as the long period waveform, and detects the current electrocardiogram waveform as the short period waveform. The control unit 14 displays the current electrocardiogram waveform and the electrocardiogram waveform one hour before side by side in the vertical direction in such a manner that the beat divided from the current electrocardiogram waveform is included in a beat time interval t12 / t13 / t14 of the electrocardiogram waveform one hour before. In this example, since a beat period of the electrocardiogram waveform one hour before is long, the current electrocardiogram waveform is displayed intermittently.

The control unit 14 may also display information indicating a start of a beat (one-dot chain lines e15 to e17 in the illustrated example) as illustrated. By referring to such display, the user can more clearly grasp a difference between shapes of the beats along with the lapse of time, a time taken for one beat, and the like.

Although not illustrated, the beats divided from the short period waveform may be displayed in an extended manner in accordance with the time interval between the beats divided from the long period waveform in the same manner as in FIG. 18.

FIG. 23B illustrates an example in which two waveforms are displayed in the overlapped manner. The control unit 14 performs the division of the beats from the current (near 15: 00: 00) electrocardiographic waveform and the electrocardiogram waveform one hour before (near 14: 00: 00), the comparison of the beat time intervals, and the detection of the long period waveform / short period waveform in the same manner as in the example of FIG. 23A. The control unit 14 displays the current electrocardiogram waveform and the electrocardiogram waveform one hour before in the overlapped manner such that the beat divided from the current electrocardiogram waveform (short period waveform) is included in a beat time interval t15 / t16 / t17 of the electrocardiogram waveform one hour before (long period waveform). In this example, since the beat period of the electrocardiogram waveform one hour before is long, the current electrocardiogram waveform is displayed intermittently. With such display, the user can still more clearly grasp the difference between the shapes of the beats along with the lapse of time, the time taken for one beat, and the like. It is also possible to clearly grasp magnitude of deviation between two beats by overlapping.

Although display examples in which the X coordinates of the starts of the beats divided from the two waveforms are the same is illustrated in the examples of FIGS. 23A and 23B, the control unit 14 may also adjust a beat arrangement position of the short period waveform within the beat time interval of the long period waveform. A detailed example is described below.

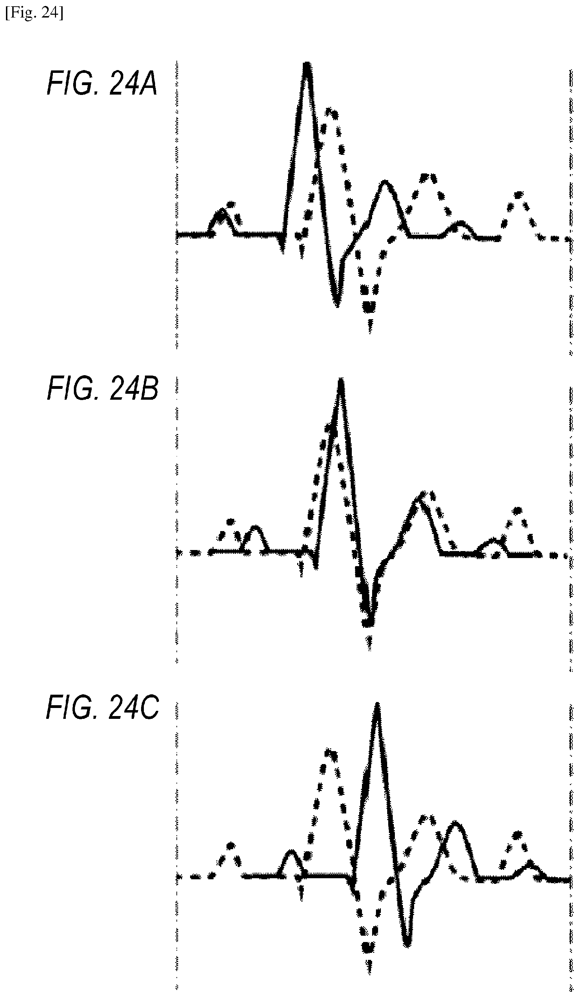

The control unit 14 performs division of beats from the two waveforms and detection of the long period waveform / short period waveform in the same manner as in the display illustrated in FIGS. 23A and 23B. Then the control unit 14 arranges the beat of the short period waveform within the time interval of the beat divided from the long period waveform at a position where a similarity between the beat divided from the long period waveform and the beat divided from the short period waveform becomes highest. An operation concept thereof will be described with reference to FIGS. 24A to 24C.

In the examples of FIGS. 24A to 24C, a waveform indicated by a dotted line is the beat divided from the short period waveform, and a waveform indicated by a solid line is the beat divided from the long period waveform. The control unit 14 fixes a position of the beat divided from the long period waveform, and shifts a horizontal axis position of the beat divided from the short period waveform. The control unit 14 fixes vertical axis positions of the two beats in such a manner that base lines thereof overlap each other.

In the example of FIG. 24A, start positions (X coordinate positions) of the waveforms of the two beats are the same. In this state, the control unit 14 detects the similarity between the two beats. For example, any similarity comparison method used in general image processing (for example, geometric pattern matching) may be used for calculation of the similarity.

After calculating the similarity, the control unit 14 shifts the horizontal axis position of the beat divided from the short period waveform. FIG. 24B illustrates an example in which the beat extracted from the short period waveform is shifted in a rightward direction. Then the control unit 14 calculates a similarity (for example, a correlation coefficient) between the two beats arranged in the same manner as in FIG. 24A.

The control unit 14 repeats the calculation of the similarity while shifting the beat divided from the short period waveform until an end point position of the beat divided from the short period waveform and an end point position of the beat extracted from the long period waveform become the same. FIG. 24C is a diagram illustrating an example in which the end point position of the beat extracted from the short period waveform and the end point position of the beat extracted from the long period waveform are the same.

The control unit 14 calculates the similarity at all the positions and determines a position where the similarity is the highest. Then the control unit 14 determines the position where the similarity is the highest as a display position of the beat divided from the short period waveform, and displays the two beats. That is, the control unit 14 adjusts the display position of the beat divided from the short period waveform based on the similarity between the beat of the long period waveform and the beat divided from the short period waveform. In the examples of FIGS. 24A to 24C, the control unit 14 determines that the arrangement position illustrated in FIG. 24B, where a degree of overlap is the highest, is the position where the similarity is the highest.

Although the similarity between the three arrangement positions is described in the example of FIGS. 24A to 24C, it is preferable to actually calculate the similarity at each position shifted by a minimum unit (for example, one pixel) or a predetermined unit.

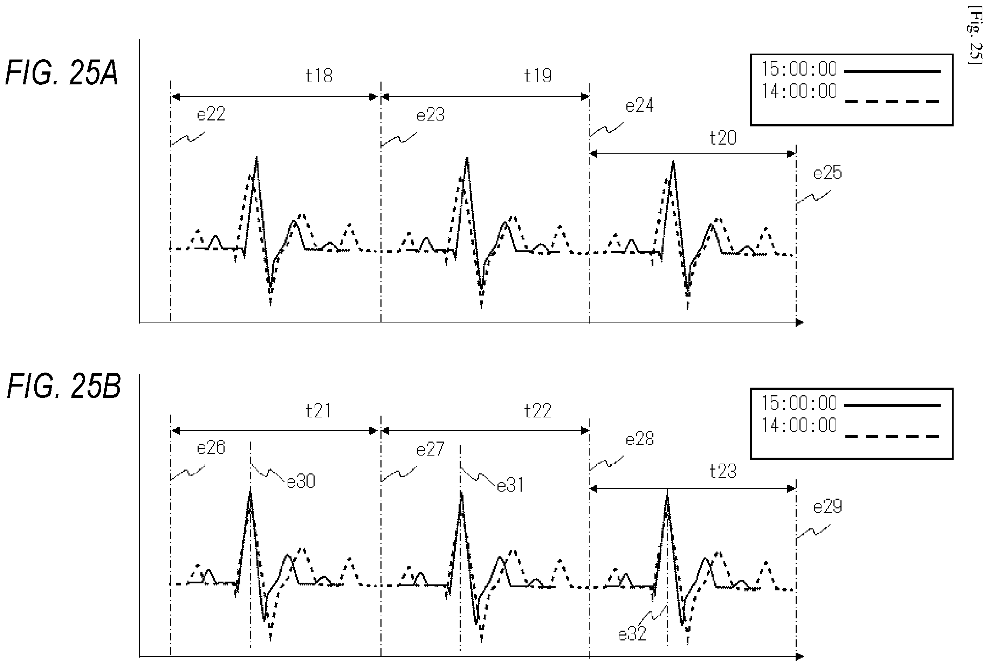

FIG. 25A is an example of such display. As in FIGS. 23A and 23B and the like, the control unit 14 divides beats from waveforms in two time zones and detects the long period waveform / short period waveform. Then the control unit 14 calculates the similarity by shifting the position of the beat divided from the short period waveform, thereby determining the arrangement position of the beat divided from the short period waveform. In the example of FIG. 25A, the control unit 14 performs display while assuming that the similarity becomes the highest at a position where there is a slight deviation between Q waves (falling points of steepest edges). Therefore, there is a deviation between a start point of a beat (a beat indicated by a solid line) divided from the short period waveform and a start point of a beat (a beat indicated by a dotted line) divided from the long period waveform. In other words, the start point of the beat divided from the short period waveform is located at a position different from start points e22 to e24 of the beat divided from the long period waveform. By referring to such display, the user can more clearly grasp a degree of deviation between shapes of the two beats.

The adjustment of the arrangement position of the beat divided from the short period waveform is not limited to the method using the similarity. For example, the control unit 14 may arrange the beat divided from the short period waveform at a position where a horizontal axis position of a Q wave of the beat divided from the long period waveform and a horizontal axis position of a Q wave of the beat divided from the short period waveform are the same. Such an example is illustrated in FIG. 25B.

The control unit 14 detects the Q wave from each beat after the beat division and the detection of the long period waveform / short period waveform. The detection of the Q wave may be performed by a method used in a general electrocardiogram analysis. The control unit 14 arranges and displays the beat divided from the short period waveform in such a manner that the horizontal axis positions of the Q waves of the two beats are the same. In the example of FIG. 25B, the display is performed in such a manner that timings e30, e31, and e32 of the Q waves of the beats divided from the two waveforms overlap each other. As a result, the start point of the beat divided from the short period waveform is located at a position different from start points e26 to e28 of the beat divided from the long period waveform.

The processing of aligning the positions of the Q waves is merely an example of arranging the feature points of the two beats at the corresponding positions, and the arrangement of the beats may also be adjusted based on other feature points, such as T waves. That is, the control unit 14 detects the feature point of the beat divided from the long period waveform and the feature point of the beat divided from the short period waveform. Then the control unit 14 displays the two beats in such a manner that the feature points of the two beats are arranged at the corresponding positions.

Although an example in which the two beats are displayed in the overlapped manner is described in FIGS. 24A to 25B, the concept is also applicable to a case where the two beats are displayed vertically side by side.

Of course, the display concepts illustrated in FIGS. 24A to 25B can also be applied to the display related to the pulsation of the brain tissue described above. That is, in the display illustrated in FIGS. 17 and 18, the control unit 14 may adjust the display position of the beat divided from the short period waveform group based on the similarity between the beat divided from the long period waveform group and the beat divided from the short period waveform group. The control unit 14 may also adjust the display position of the beat divided from the short period waveform group within the beat time interval of the long period waveform group in such a manner that the feature point of the beat divided from the long period waveform group and the feature point of the beat divided from the short period waveform group are arranged at the corresponding positions during the display illustrated in FIGS. 17 and 18.

Of course, the display example described above is also applicable to display of other vital signs (display of a respiratory waveform at the first time and a respiratory waveform at the second time, display of a blood pressure waveform at the first time and a blood pressure waveform at the second time, display of an arterial oxygen saturation waveform at the first time and an arterial oxygen saturation waveform at the second time, and the like). Such a display concept can also be interpreted as in the following supplementary notes.

### Supplementary Note 1

A physiological information processing device includes: an acquisition unit that acquires a physiological signal from a sensor attached to a subject at a first time and a second time; and
a control unit that detects a feature point of a first physiological waveform based on the physiological signal at the first time and a feature point of a second physiological waveform based on the physiological signal at the second time, the second physiological waveform being a physiological waveform of the same type as the first physiological waveform, arranges the feature point of the first physiological waveform and the feature point of the second physiological waveform at corresponding positions, and displays the first physiological waveform and the second waveform.

### Supplementary Note 2

The patient monitor according to supplementary note 1 is characterized in that the control unit displays information indicating the corresponding positions.

### Supplementary Note 3

A patient monitor includes: an acquisition unit that acquires a physiological signal from a sensor attached to a subject at a first time and a second time; and
a control unit that divides a first physiological waveform into beats based on the physiological signal at the first time, and divides a second physiological waveform into beats based on the physiological signal at the second time, the second physiological waveform being a physiological waveform of the same type as the first physiological waveform,
compares a beat time interval of the first waveform with a beat time interval of the second waveform, and detects one of the first waveform and the second waveform whose time interval is longer as a long period waveform and the other whose time interval is shorter as a short period waveform, and
displays the first waveform and the second waveform in such a manner that a beat divided from the short period waveform is included within the beat time interval of the long period waveform.

### Supplementary Note 4

The patient monitor according to supplementary note 3 is characterized in that the control unit adjusts a display position of the beat divided from the short period waveform within the beat time interval of the long period waveform based on a similarity between the beat divided from the long period waveform and the beat divided from the short period waveform.

### Supplementary Note 5

The patient monitor according to supplementary note 3 is characterized in that the control unit detects a beat feature point of the long period waveform and a beat feature point of the short period waveform, respectively, and adjusts a display position of the beat divided from the short period waveform within the beat time interval of the long period waveform in such a manner that the feature points are arranged at corresponding positions.

For example, although the vertical axis indicates the body inside position (depth) while the horizontal axis indicates the information on the elapsed time (time, time from the start of measurement, and the like) in the examples described above, the vertical axis may indicate the information on the elapsed time while the horizontal axis indicates the body inside position (depth).

This application claims priority to Japanese Patent Application No. 2020-056709 filed on March 26, 2020.

### [Industrial Applicability]

According to the present disclosed subject matter, there is provided a patient monitor, a physiological information display method, and a program that provide the screen by which the relationship between the depth and the motion in the body of the subject can be easily grasped.

## Claims

1. A patient monitor (10) comprising:
an acquisition unit (11), which is configured to acquire a reflected wave indicating a motion of a brain tissue generated at each depth by an ultrasonic wave irradiated at a plurality of depths in a body of a subject; and
a control unit (14), which is configured to display, in terms of the following expression A),
a change over time in an index value of the motion of the brain tissue at each depth of the subject calculated from the reflected wave:
A) map information expressing a magnitude of the index value on a map taking the depth on a first axis and elapsed time on a second axis, by a change in at least one of hue, lightness, and chroma.

2. The patient monitor (10) according to claim 1, wherein the control unit (14) is configured to perform a comparison using the index value of each depth at a first time and the index value of each depth at a second time, and perform a notification when it is determined, based on the comparison, that a change in the index value is large along with a lapse of time.

3. The patient monitor (10) according to claim 2, wherein the control unit (14) is configured to perform the notification by at least one method among sounding of an alarm sound, displaying of a message, and automatic switching of a screen.

4. The patient monitor (10) according to claim 2 or 3, wherein the control unit (14) is configured to display a change over time in the index value of each depth at the first time and a change over time in the index value of each depth at the second time.

5. The patient monitor (10) according to claim 4, wherein the control unit (14) is configured to display the change over time in the index value of each depth at the first time and the change over time in the index value of each depth at the second time in an overlapping manner in terms of a plurality of waveforms corresponding to each depth.

6. The patient monitor (10) according to claim 4 or 5, wherein the control unit (14) is configured to display in such a manner that a display mode of a waveform indicating the first time and a display mode of a waveform indicating the second time are different from each other.

7. The patient monitor (10) according to any one of claims 1 to 6, wherein the control unit (14) is configured to use the index value of each depth to perform a mutual comparison so as to determine whether there is any depth having a large difference from surroundings, and perform a notification when there is a depth having a large difference from the surroundings based on the determination.

8. The patient monitor (10) according to claim 7, wherein the control unit (14) is configured to use the index value at a first depth and the index value at a second depth which is a position shallower than the first depth to perform the comparison and uses the index value at the first depth and the index value at a third depth which is a position deeper than the first depth to perform the comparison, so as to determine whether the first depth is the depth whose index value has a large difference from the surroundings.

9. The patient monitor (10) according to any one of claims 1 to 8, wherein the control unit (14) is configured to display a plurality of waveforms corresponding to each depth in an overlapping manner on the map information related to A).

10. The patient monitor (10) according to any one of claims 1 to 9, wherein the control unit (14) is configured to acquire a specified depth which is set by a user as a display target, calculate the index value corresponding to the specified depth, and use the calculated index value to display a change over time in the index value of the specified depth.

11. The patient monitor (10) according to any one of claims 1 to 9, wherein the control unit (14) is configured to acquire a specified time which is set by a user as a display target, calculate the index value corresponding to the specified time, and use the calculated index value to display a change over time in the index value of the specified time.

12. The patient monitor (10) according to any one of claims 1 to 11, wherein the control unit (14) is configured to change and display a display mode of a waveform at each depth in accordance with magnitude of the index value at each depth.

13. The patient monitor (10) according to any one of claims 2 to 12, wherein the control unit (14) is configured to:
detect a feature point of a first waveform group which is a plurality of waveforms indicating the change over time in the index value of each depth at the first time and a feature point of a second waveform group which is a plurality of waveforms indicating the change over time in the index value of each depth at the second time, and
arrange the feature point of the first waveform group and the feature point of the second waveform group at corresponding positions, and display the first waveform group and the second waveform group.

14. The patient monitor (10) according to any one of claims 2 to 12, wherein the control unit (14) is configured to:
divide, for each beat, a first waveform group which is a plurality of waveforms indicating a change over time in the index value of each depth at the first time, and divide, for each beat, a second waveform group which is a plurality of waveforms indicating a change over time in the index value of each depth at the second time,
compare a beat time interval of the first waveform group with a beat time interval of the second waveform group, and detect one of the first waveform group and the second waveform group whose time interval is longer as a long period waveform group and the other whose time interval is shorter as a short period waveform group, and
display the first waveform group and the second waveform group in such a manner that a beat divided from the short period waveform group is included within the beat time interval of the long period waveform group.

15. The patient monitor (10) according to claim 14, wherein the control unit (14) is configured to adjust a display position of the beat divided from the short period waveform group within the beat time interval of the long period waveform group based on a similarity between a beat divided from the long period waveform group and the beat divided from the short period waveform group, or a feature point of the beat divided from the long period waveform group and a feature point of the beat divided from the short period waveform group.

16. A patient monitor (10) comprising:
an acquisition unit (11), which is configured to acquire a first reflected wave indicating a motion of a brain tissue generated at a first depth by an ultrasonic wave radiated to the first depth in a body of a subject, and a second reflected wave indicating a motion generated at a second depth by an ultrasonic wave radiated to the second depth in the body of the subject; and
a control unit (14), which is configured to display, in terms of the following expression A),
changes over time in both a first index value of the brain tissue calculated from the first reflected wave and a second index value calculated from the second reflected wave:
A) map information expressing a magnitude of the first and second index values on a map taking the depth on a first axis and elapsed time on a second axis, by a change in at least one of hue, lightness, and chroma.

17. An ultrasonic wave processing method comprising:
an acquisition step of acquiring a reflected wave indicating a motion of a brain tissue generated at each depth by an ultrasonic wave irradiated at a plurality of depths in a body of a subject; and
a control step of displaying, in terms of the following expression A), a change over time in an index value of the motion of the brain tissue at each depth of the subject calculated from the reflected wave:
A) map information expressing a magnitude of the index value on a map taking the depth on a first axis and elapsed time on a second axis, by a change in at least one of hue, lightness, and chroma.

18. A program which causes a computer to execute:
an acquisition step of acquiring a reflected wave indicating a motion of a brain tissue generated at each depth by an ultrasonic wave irradiated at a plurality of depths in a body of a subject, and
a control step of displaying, in terms of the following expression A), a change over time in an index value of the motion of the brain tissue at each depth of the subject calculated from the reflected wave:
A) map information expressing a magnitude of the index value on a map taking the depth on a first axis and elapsed time on a second axis, by a change in at least one of hue, lightness, and chroma.

## Patentansprüche

1. Patienten-Überwachungsvorrichtung (10), die umfasst:
eine Erfassungs-Einheit (11), die so ausgeführt ist, dass sie eine reflektierte Welle erfasst, die eine Bewegung eines Gehirngewebes angibt, die in jeder Tiefe von einer Ultraschallwelle erzeugt wird, die in einer Vielzahl von Tiefen in einem Körper eines Patienten ausgesendet wird; sowie
eine Steuerungs-Einheit (14), die so ausgeführt ist, dass sie entsprechend dem folgenden Ausdruck A) eine Änderung eines Indexwertes der Bewegung des Gehirngewebes in jeder Tiefe des Patienten im Verlauf der Zeit anzeigt, die aus der reflektierten Welle berechnet wird:
A) Abbildungsinformationen, die einen Betrag des Indexwertes in einer Abbildung, in der die Tiefe auf einer ersten Achse und verstrichene Zeit auf einer zweiten Achse dargestellt werden, anhand einer Änderung von Farbton, Helligkeit oder/und Sättigung ausdrücken.

2. Patienten-Überwachungsvorrichtung (10) nach Anspruch 1, wobei die Steuerungs-Einheit (14) so ausgeführt ist, dass sie einen Vergleich unter Verwendung des Indexwertes jeder Tiefe zu einem ersten Zeitpunkt und des Indexwertes jeder Tiefe zu einem zweiten Zeitpunkt durchführt und eine Mitteilung macht, wenn auf Basis des Vergleichs festgestellt wird, dass eine Änderung des Indexwertes mit einem Verstreichen von Zeit groß ist.

3. Patienten-Überwachungsvorrichtung (10) nach Anspruch 2, wobei die Steuerungs-Einheit (14) so ausgeführt ist, dass sie die Mitteilung mittels wenigstens eines Verfahrens vom Ertönen eines Warntons, Anzeigen einer Nachricht oder/und automatischen Umschalten eines Bildschirms macht.

4. Patienten-Überwachungsvorrichtung (10) nach Anspruch 2 oder 3, wobei die Steuerungs-Einheit (14) so ausgeführt ist, dass sie eine Änderung des Indexwertes jeder Tiefe im Verlauf der Zeit zu dem ersten Zeitpunkt und eine Änderung des Indexwertes jeder Tiefe im Verlauf der Zeit zu dem zweiten Zeitpunkt anzeigt.

5. Patienten-Überwachungsvorrichtung (10) nach Anspruch 4, wobei die Steuerungs-Einheit (14) so ausgeführt ist, dass sie die Änderung des Indexwertes jeder Tiefe im Verlauf der Zeit zu dem ersten Zeitpunkt und eine Änderung des Indexwertes jeder Tiefe im Verlauf der Zeit zu dem zweiten Zeitpunkt überlappend als eine Vielzahl jeder Tiefe entsprechender Wellenformen anzeigt.

6. Patienten-Überwachungsvorrichtung (10) nach Anspruch 4 oder 5, wobei die Steuerungs-Einheit (14) so ausgeführt ist, dass sie Anzeige so durchführt, dass ein Anzeigemodus einer Wellenform, die den ersten Zeitpunkt angibt, und ein Anzeigemodus einer Wellenform, die den zweiten Zeitpunkt angibt, sich voneinander unterscheiden.

7. Patienten-Überwachungsvorrichtung (10) nach einem der Ansprüche 1 bis 6, wobei die Steuerungs-Einheit (14) so ausgeführt ist, dass sie den Indexwert jeder Tiefe nutzt, um einen wechselseitigen Vergleich durchzuführen und festzustellen, ob es eine Tiefe mit einer großen Differenz gegenüber der Umgebung gibt, und eine Mitteilung macht, wenn es basierend auf der Feststellung eine Tiefe mit einer großen Differenz gegenüber der Umgebung gibt.

8. Patienten-Überwachungsvorrichtung (10) nach Anspruch 7, wobei die Steuerungs-Einheit (14) so ausgeführt ist, dass sie den Indexwert in einer ersten Tiefe und den Indexwert in einer zweiten Tiefe, die an einer Position liegt, die flacher ist als die erste Tiefe, nutzt, um den Vergleich durchzuführen, und den Indexwert in der ersten Tiefe und den Indexwert in einer dritten Tiefe, die an einer Position liegt, die tiefer ist als die erste Tiefe, nutzt, um den Vergleich durchzuführen und festzustellen, ob die erste Tiefe die Tiefe ist, deren Indexwert eine große Differenz gegenüber der Umgebung aufweist.

9. Patienten-Überwachungsvorrichtung (10) nach einem der Ansprüche 1 bis 8, wobei die Steuerungs-Einheit (14) so ausgeführt ist, dass sie eine Vielzahl von Wellenformen, die jeder Tiefe entsprechen, überlappend in den Abbildungsinformationen entsprechend A) anzeigt.

10. Patienten-Überwachungsvorrichtung (10) nach einem der Ansprüche 1 bis 9, wobei die Steuerungs-Einheit (14) so ausgeführt ist, dass sie eine vorgegebene Tiefe erfasst, die von einem Benutzer als ein Anzeige-Ziel festgelegt wird, den Indexwert entsprechend der vorgegebenen Tiefe berechnet und den berechneten Indexwert nutzt, um eine Änderung des Indexwertes der vorgegebenen Tiefe im Verlauf der Zeit anzuzeigen.

11. Patienten-Überwachungsvorrichtung (10) nach einem der Ansprüche 1 bis 9, wobei die Steuerungs-Einheit (14) so ausgeführt ist, dass sie einen vorgegebenen Zeitpunkt erfasst, der von einem Benutzer als ein Anzeige-Ziel festgelegt wird, den Indexwert entsprechend dem vorgegebenen Zeitpunkt berechnet und den berechneten Indexwert nutzt, um eine Änderung des Indexwertes des vorgegebenen Zeitpunktes im Verlauf der Zeit anzuzeigen.

12. Patienten-Überwachungsvorrichtung (10) nach einem der Ansprüche 1 bis 11, wobei die Steuerungs-Einheit (14) so ausgeführt ist, dass sie einen Anzeigemodus einer Wellenform an jeder Tiefe entsprechend dem Betrag des Indexwertes an jeder Tiefe ändert und anzeigt.

13. Patienten-Überwachungsvorrichtung (10) nach einem der Ansprüche 2 bis 12, wobei die Steuerungs-Einheit (14) so ausgeführt ist, dass sie durchführt:
Erfassen eines Merkmalspunktes einer ersten Wellenform-Gruppe, die eine Vielzahl von Wellenformen ist, die die Änderung des Indexwertes jeder Tiefe im Verlauf der Zeit zu dem ersten Zeitpunkt angibt, und eines Merkmalspunktes einer zweiten Wellenform-Gruppe, die eine Vielzahl von Wellenformen ist, die die Änderung des Indexwertes jeder Tiefe im Verlauf der Zeit zu dem zweiten Zeitpunkt angibt, und
Anordnen des Merkmalspunktes der ersten Wellenform-Gruppe und des Merkmalspunktes der zweiten Wellenform-Gruppe an entsprechenden Positionen und Anzeigen der ersten Wellenform-Gruppe sowie der zweiten Wellenform-Gruppe.

14. Patienten-Überwachungsvorrichtung (10) nach einem der Ansprüche 2 bis 12, wobei die Steuerungs-Einheit (14) so ausgeführt ist, dass sie durchführt:
Abteilen einer ersten Wellenform-Gruppe, die eine Vielzahl von Wellenformen ist, die eine Änderung des Indexwertes jeder Tiefe im Verlauf der Zeit zu dem ersten Zeitpunkt anzeigt, für jeden Schlag, sowie
Abteilen einer zweiten Wellenform-Gruppe, die eine Vielzahl von Wellenformen ist, die eine Änderung des Indexwertes jeder Tiefe im Verlauf der Zeit zu dem zweiten Zeitpunkt angibt, für jeden Schlag,
Vergleichen eines Schlag-Zeitintervalls der ersten Wellenform-Gruppe mit einem Schlag-Zeitintervall der zweiten Wellenform-Gruppe, sowie
Erfassen der ersten Wellenform-Gruppe oder der zweiten Wellenform-Gruppe, deren Zeitintervall länger ist, als eine Wellenform-Gruppe mit langer Periode, und der anderen, deren Zeitintervall kürzer ist, als eine Wellenform-Gruppe mit kurzer Periode, sowie Anzeigen der ersten Wellenform-Gruppe und der zweiten Wellenform-Gruppe in einer Form, bei der ein von der Wellenform-Gruppe mit kurzer Periode abgeteilter Schlag in dem Schlag-Zeitintervall der Wellenform-Gruppe mit langer Periode enthalten ist.

15. Patienten-Überwachungsvorrichtung (10) nach Anspruch 14, wobei die Steuerungs-Einheit (14) so ausgeführt ist, dass sie eine Anzeigeposition des von der Wellenform-Gruppe mit kurzer Periode abgeteilten Schlags innerhalb des Schlag-Zeitintervalls der Wellenform-Gruppe mit langer Periode auf Basis einer Ähnlichkeit zwischen einem von der Wellenform-Gruppe mit langer Periode abgeteilten Schlag und dem von der Wellenform-Gruppe mit kurzer Periode abgeteilten Schlag oder eines Merkmalspunktes des von der Wellenform-Gruppe mit langer Periode abgeteilten Schlags und eines Merkmalspunktes des von der Wellenform-Gruppe mit kurzer Periode abgeteilten Schlags anpasst.

16. Patienten-Überwachungsvorrichtung (10), die umfasst:
eine Erfassungs-Einheit (11), die so ausgeführt ist, dass sie eine erste reflektierte Welle, die eine Bewegung eines Gehirngewebes angibt, die in einer ersten Tiefe von einer Ultraschallwelle erzeugt wird, die in einer ersten Tiefe in einem Körper eines Patienten ausgesendet wird, und eine zweite reflektierte Welle erfasst, die eine Bewegung anzeigt, die in einer zweiten Tiefe von einer Ultraschallwelle erzeugt wird, die in der zweiten Tiefe in dem Körper des Patienten ausgesendet wird; sowie
eine Steuerungs-Einheit (14), die so ausgeführt ist, dass sie entsprechend dem folgenden Ausdruck A) Änderungen sowohl eines ersten Indexwertes des Gehirngewebes, der aus der ersten reflektierten Welle berechnet wird, als auch eines zweiten Indexwertes anzeigt, die aus der zweiten reflektierten Welle berechnet wird;
A) Abbildungsinformationen, die einen Betrag des ersten und des zweiten Indexwertes in einer Abbildung, in der die Tiefe auf einer ersten Achse und verstrichene Zeit auf einer zweiten Achse dargestellt werden, anhand einer Änderung von Farbton, Helligkeit oder/und Sättigung ausdrücken.

17. Verfahren zum Verarbeiten von Ultraschallwellen, das umfasst:
einen Erfassungs-Schritt, in dem eine reflektierte Welle erfasst wird, die eine Bewegung eines Gehirngewebes anzeigt, die in jeder Tiefe von einer Ultraschallwelle erzeugt wird, die in einer Vielzahl von Tiefen in einem Körper eines Patienten ausgesendet wird; sowie einen Steuerungs-Schritt, in dem entsprechend dem folgenden Ausdruck A) eine Änderung eines Indexwertes der Bewegung des Gehirngewebes in jeder Tiefe des Patienten im Verlauf der Zeit angezeigt wird, die aus der reflektierten Welle berechnet wird:
A) Abbildungsinformationen, die einen Betrag des Indexwertes in einer Abbildung, in der die Tiefe auf einer ersten Achse und verstrichene Zeit auf einer zweiten Achse dargestellt werden, anhand einer Änderung von Farbton, Helligkeit oder/und Sättigung ausdrücken.

18. Programm, das einen Computer veranlasst, auszuführen:
einen Erfassungs-Schritt, in dem eine reflektierte Welle erfasst wird, die eine Bewegung eines Gehirngewebes anzeigt, die in jeder Tiefe von einer Ultraschallwelle erzeugt wird, die in einer Vielzahl von Tiefen in einem Körper eines Patienten ausgesendet wird; sowie einen Steuerungs-Schritt, in dem entsprechend dem folgenden Ausdruck A) eine Änderung eines Indexwertes der Bewegung des Gehirngewebes in jeder Tiefe des Patienten im Verlauf der Zeit angezeigt wird, die aus der reflektierten Welle berechnet wird:
A) Abbildungsinformationen, die einen Betrag des Indexwertes in einer Abbildung, in der die Tiefe auf einer ersten Achse und verstrichene Zeit auf einer zweiten Achse dargestellt werden, anhand einer Änderung von Farbton, Helligkeit oder/und Sättigung ausdrücken.

## Revendications

1. Dispositif de surveillance d'un patient (10) comprenant :
une unité d'acquisition (11), qui est configurée pour acquérir une onde reflétée indiquant un mouvement d'un tissu cérébral généré à chaque profondeur par une onde ultrasonore irradiée à une pluralité de profondeurs dans un corps d'un sujet ; et
une unité de commande (14), qui est configurée pour afficher, en termes de l'expression suivante A), un changement dans le temps dans une valeur d'indice du mouvement du tissu cérébral à chaque profondeur du sujet calculée à partir de l'onde reflétée :
A) une information cartographique exprimant une magnitude de la valeur d'indice sur une carte prenant la profondeur sur un premier axe et un temps écoulé sur un second axe, par un changement dans au moins l'une d'une teinte, d'une luminosité, et d'une saturation.

2. Dispositif de surveillance d'un patient (10) selon la revendication 1, dans lequel l'unité de commande (14) est configurée pour exécuter une comparaison en utilisant la valeur d'indice de chaque profondeur à un premier moment et la valeur d'indice de chaque profondeur à un second moment, et pour effectuer une notification lorsqu'il est déterminé, sur la base de la comparaison, qu'un changement dans la valeur d'indice est grand durant un laps de temps.

3. Dispositif de surveillance d'un patient (10) selon la revendication 2, dans lequel l'unité de commande (14) est configurée pour effectuer la notification selon au moins un procédé parmi l'envoi d'un son d'alarme, l'affichage d'un message, et un basculement automatique d'un écran.

4. Dispositif de surveillance d'un patient (10) selon la revendication 2 ou 3, dans lequel l'unité de commande (14) est configurée pour afficher un changement dans le temps dans la valeur d'indice de chaque profondeur au premier moment et un changement dans le temps dans la valeur d'indice de chaque profondeur au second moment.

5. Dispositif de surveillance d'un patient (10) selon la revendication 4, dans lequel l'unité de commande (14) est configurée pour afficher le changement dans le temps dans la valeur d'indice de chaque profondeur au premier moment et le changement dans le temps dans la valeur d'indice de chaque profondeur au second moment d'une manière chevauchante en termes de pluralité de formes d'onde correspondant à chaque profondeur.

6. Dispositif de surveillance d'un patient (10) selon la revendication 4 ou 5, dans lequel l'unité de commande (14) est configurée pour l'affichage d'une manière telle qu'un mode d'affichage d'une forme d'onde indiquant le premier moment et qu'un mode d'affichage d'une forme d'onde indiquant le second moment sont différents l'un de l'autre.

7. Dispositif de surveillance d'un patient (10) selon l'une quelconque des revendications 1 à 6, dans lequel l'unité de commande (14) est configurée pour utiliser la valeur d'indice de chaque profondeur pour effectuer une comparaison mutuelle afin de déterminer s'il existe une quelconque profondeur ayant une grande différence parmi les endroits environnants, et effectuer une notification lorsqu'il existe une profondeur ayant une grande différence parmi les endroits environnants sur la base de la détermination.

8. Dispositif de surveillance d'un patient (10) selon la revendication 7, dans lequel l'unité de commande (14) est configurée pour utiliser la valeur d'indice à une première profondeur et la valeur d'indice à une seconde profondeur qui est une position moins profonde que la première profondeur pour effectuer la comparaison et qui utilise la valeur d'indice à la première profondeur et la valeur d'indice à une troisième profondeur qui est une position plus profonde que la première profondeur pour effectuer la comparaison, afin de déterminer si la première profondeur est la profondeur dont la valeur d'indice présente une grande différence parmi les endroits environnants.

9. Dispositif de surveillance d'un patient (10) selon l'une quelconque des revendications 1 à 8, dans lequel l'unité de commande (14) est configurée pour afficher une pluralité de formes d'onde correspondant à chaque profondeur d'une manière chevauchante sur l'information cartographique liée à A).

10. Dispositif de surveillance d'un patient (10) selon l'une quelconque des revendications 1 à 9, dans lequel l'unité de commande (14) est configurée pour acquérir une profondeur spécifiée qui est définie par un utilisateur comme cible d'affichage, calculer la valeur d'indice correspondant à la profondeur spécifiée, et utiliser la valeur d'indice calculée pour afficher un changement dans le temps dans la valeur d'indice de la profondeur spécifiée.

11. Dispositif de surveillance d'un patient (10) selon l'une quelconque des revendications 1 à 9, dans lequel l'unité de commande (14) est configurée pour acquérir un moment spécifié qui est défini par un utilisateur comme cible d'affichage, calculer la valeur d'indice correspondant au moment spécifié, et utiliser la valeur d'indice calculée pour afficher un changement dans le temps dans la valeur d'indice du moment spécifié.

12. Dispositif de surveillance d'un patient (10) selon l'une quelconque des revendications 1 à 11, dans lequel l'unité de commande (14) est configurée pour changer et afficher un mode d'affichage d'une forme d'onde à chaque profondeur selon une magnitude de la valeur d'indice à chaque profondeur.

13. Dispositif de surveillance d'un patient (10) selon l'une quelconque des revendications 2 à 12, dans lequel l'unité de commande (14) est configurée pour :
détecter un point caractéristique d'un premier groupe de formes d'onde qui est une pluralité de formes d'onde indiquant le changement dans le temps dans la valeur d'indice de chaque profondeur au premier moment et un point caractéristique d'un second groupe de formes d'onde qui est une pluralité de formes d'onde indiquant le changement dans le temps dans la valeur d'indice de chaque profondeur au second moment, et
agencer le point caractéristique du premier groupe de formes d'onde et le point caractéristique du second groupe de formes d'onde à des positions correspondantes, et afficher le premier groupe de formes d'onde et le second groupe de formes d'onde.

14. Dispositif de surveillance d'un patient (10) selon l'une quelconque des revendications 2 à 12, dans lequel l'unité de commande (14) est configurée pour :
séparer, pour chaque battement, un premier groupe de formes d'onde qui est une pluralité de formes d'onde indiquant un changement dans le temps dans la valeur d'indice de chaque profondeur au premier moment, et séparer, pour chaque battement, un second groupe de formes d'onde qui est une pluralité de formes d'onde indiquant un changement dans le temps dans la valeur d'indice de chaque profondeur au second moment,
comparer un intervalle de temps de battement du premier groupe de formes d'onde à un intervalle de temps de battement du second groupe de formes d'onde, et détecter l'un du premier groupe de formes d'onde et du second groupe de formes d'onde dont l'intervalle de temps est plus long comme un groupe de formes d'onde de longue période et l'autre dont l'intervalle de temps est plus court comme groupe de formes d'onde de période courte, et
pour afficher le premier groupe de formes d'onde et le second groupe de formes d'onde d'une manière telle qu'un battement séparé du groupe de formes d'onde de période courte est inclus à l'intérieur de l'intervalle de temps de battement du groupe de formes d'onde de longue période.

15. Dispositif de surveillance d'un patient (10) selon la revendication 14, dans lequel l'unité de commande (14) est configurée pour ajuster une position d'affichage du battement séparé du groupe de formes d'onde de période courte à l'intérieur de l'intervalle de temps de battement du groupe de formes d'onde de longue période sur la base d'une similitude entre un battement séparé du groupe de formes d'onde de longue période et le battement séparé du groupe de formes d'onde de période courte, ou un point caractéristique du battement séparé du groupe de formes d'onde de longue période et un point caractéristique du battement séparé du groupe de formes d'onde de période courte.

16. Dispositif de surveillance d'un patient (10) comprenant :
une unité d'acquisition (11), qui est configurée pour acquérir une première onde reflétée indiquant un mouvement d'un tissu cérébral généré à une première profondeur par une onde ultrasonore irradiée vers la première profondeur dans un corps d'un sujet, et une seconde onde reflétée indiquant un mouvement généré à une seconde profondeur par une onde ultrasonore irradiée vers la seconde profondeur dans le corps du sujet ; et
une unité de commande (14), qui est configurée pour afficher, en termes de l'expression suivante A), des changements dans le temps dans à la fois une première valeur d'indice du tissu cérébral calculée à partir de la première onde reflétée et une seconde valeur d'indice calculée à partir de la seconde onde reflétée :
A) une information cartographique exprimant une magnitude de la première et de la seconde valeur d'indice sur une carte prenant la profondeur sur un premier axe et un temps écoulé sur un second axe, par un changement dans au moins l'une d'une teinte, d'une luminosité, et d'une saturation.

17. Procédé de traitement d'onde ultrasonore comprenant :
une étape d'acquisition consistant à acquérir une onde reflétée indiquant un mouvement d'un tissu cérébral généré à chaque profondeur par une onde ultrasonore irradiée à une pluralité de profondeurs dans un corps d'un sujet ; et
une étape de commande d'affichage, en termes de l'expression suivante A), un changement dans le temps dans une valeur d'indice du mouvement du tissu cérébral à chaque profondeur du sujet calculée à partir de l'onde reflétée :
A) une information cartographique exprimant une magnitude de la valeur d'indice sur une carte prenant la profondeur sur un premier axe et un temps écoulé sur un second axe, par un changement dans au moins l'une d'une teinte, d'une luminosité, et d'une saturation.

18. Programme qui amène un ordinateur à exécuter :
une étape d'acquisition consistant à acquérir une onde reflétée indiquant un mouvement d'un tissu cérébral généré à chaque profondeur par une onde ultrasonore irradiée à une pluralité de profondeurs dans un corps d'un sujet, et
une étape de commande d'affichage, en termes de l'expression suivante A), un changement dans le temps dans une valeur d'indice du mouvement du tissu cérébral à chaque profondeur du sujet calculée à partir de l'onde reflétée :
A) une information cartographique exprimant une magnitude de la valeur d'indice sur une carte prenant la profondeur sur un premier axe et un temps écoulé sur un second axe, par un changement dans au moins l'une d'une teinte, d'une luminosité, et d'une saturation.
